# EUROPEAN PATENT APPLICATION

(11) **EP 4 844 062 A1**
(43) Date of publication of application: **25.11.2026**
(21) Application number: 25741623.0
(22) Date of filing: 17.01.2025
(51) Int. Cl.: C12N 15/113, C12N 15/11, A61K 48/00, A61P 21/00, A61P 3/10, A61P 13/12, A61P 9/10, A61P 25/28, A61P 35/00, A61P 43/00

(54) **NUCLEIC ACID FOR INHIBITING THE EXPRESSION OF ACTIVIN RECEPTOR TYPE IIB GENE AND USE THEREOF**

(30) Priority: 19.01.2024 CN 202410084304
(71) Applicant: YUN HO BIO CO., LTD, Shanghai 201206 (CN)
(72) Inventor: ZHU, Yun, Shanghai 201206 (CN); WU, Yun, Shanghai 201206 (CN); LIU, Yang, Shanghai 201206 (CN); MAO, Song, Shanghai 201206 (CN); DONG, Xianglei, Shanghai 201206 (CN)
(74) Representative: Simmons & Simmons LLP (Munich)
(86) International application number: PCT/CN2025/073015
(87) International publication number: WO 2025/153061

(57) **Abstract**

Disclosed in the present invention are an antisense oligonucleotide or RNAi agent for inhibiting the expression of the ACVR2B gene in cells and a pharmaceutical composition thereof. The present invention also relates to the therapeutic use of the antisense oligonucleotide and RNAi agent.

## Description

### Technical Filed

The present invention relates to nucleic acids that inhibit the expression of activin receptor type IIB (ACVR2b) gene in cells, including antisense oligonucleotides (ASO) and RNAi agents (such as siRNA, and pharmaceutical compositions comprising the same. The invention also relates to therapeutic uses of the nucleic acids.

### Background

Activin type IIB receptor (ACVR2B) is a type II serine/threonine kinase receptor that belongs to the transforming growth factor-β (TGF-β) superfamily. This superfamily includes secreted polypeptide signaling molecules such as TGF-β, activins, inhibins (INH), bone morphogenetic proteins (BMPs), growth differentiation factors (GDFs), myostatin (MSTN), follistatin (FST), and the Smad protein family. The mechanism of TGF-β signal transduction involves the binding of TGF-β superfamily members to ACVR2B on the cell surface, followed by recruitment of activin type I receptors (ActRI). The resulting ligand-receptor complex activates ActRI kinases (ALK4/5), leading to phosphorylation of the Smad protein family (primarily Smad2 and Smad3) transcription factors. These phosphorylated Smads then form complexes with Smad4 and translocate into the nucleus, where they regulate the target genes of various transcription factors, thereby modulating proliferation, differentiation, migration, and apoptosis of diverse cell types. The TGF-β signaling pathway plays a critical role in embryonic development, skeletal formation, and muscle growth and development. Studies have revealed that hereditary deficiency of the key gene *Dystrophin* in mouse models of Duchenne muscular dystrophy (DMD), a clinically refractory disease, is accompanied by a marked up-regulation in TGF-β expression; similarly, hereditary deficiency of the key gene *FBN1* in mouse models of Marfan syndrome (MFS) is also associated with significantly elevated TGF-β expression. In both models, skeletal muscles exhibit impaired regenerative capacity following injury; however, upon blockade of TGF-β pathway signaling, the skeletal muscles regain their repair and regeneration functions.

Muscle atrophy is a debilitating and life-threatening pathological condition that is associated with the onset of various chronic, neurological, hereditary, inflammatory, fibrotic, or infectious disorders, including muscular dystrophy, amyotrophic lateral sclerosis (ALS), myositis, cancer, rheumatoid arthritis, osteoarthritis, diabetes mellitus, sarcopenic obesity, androgen deficiency, corticosteroid myopathy, inflammatory bowel disease, cirrhosis, chronic obstructive pulmonary disease, pulmonary fibrosis, chronic kidney disease, trauma, cardiomyopathy, chronic heart failure, and HIV infection. The SMAD signaling cascade downstream of ACVR2B regulates multiple myogenic genes, such as myogenic determination factor (MyoD), myogenin, and myogenic factor 5 (Myf5), which are involved in cellular hypertrophy, proliferation, or differentiation. Studies have demonstrated that inhibition of ACVR2B leads to rapid and substantial muscle growth, and thus it is frequently employed in the treatment of diseases such as muscle atrophy and muscular dystrophy.

Studies have demonstrated that blocking of ACVR2B-mediated MSTN pathway signaling reduces total body fat mass in mice, improves glucose metabolism, increases insulin sensitivity and energy expenditure, and even promotes the browning of white adipose tissue, thereby exerting systemic anti-diabetic and anti-obesity effects. Moreover, accumulating evidence indicates that the MSTN/activin pathway not only affects the cardiovascular system, but also impacts vascular remodeling, cellular senescence, and fibrosis. Therefore, inhibition of the MSTN pathway may play a therapeutic role in cachexia, inflammation, vascular injury, and possibly progressive renal dysfunction in patients with chronic kidney disease (CKD).

Among the numerous ligands of ACVR2B, activin A promotes the synthesis and secretion of gonadotropins by the pituitary gland to regulate reproductive physiological activities; in addition to its expression in reproductive organs, it is also present in other organs and systems, such as the brain, liver, lung, kidney, bone, and intestine. Activin A is expressed during renal development and plays an important role in kidney development and repair processes; it causes delayed branching of the ureteric bud in mice and reduces the size of cultured mouse metanephros in vitro, acting as a negative regulator of kidney development and participating in renal pathological processes such as renal fibrosis. Elevated activin A levels in chronic kidney disease (CKD) can lead to anemia and promote the progression of renal fibrosis, thereby aggravating disease progression. Furthermore, activin A can exacerbate renal injury caused by diseases such as systemic lupus erythematosus, vasculitis, and multiple myeloma (MM). Lowering activin A levels through inhibition of ACVR2A and ACVR2B can prevent acute kidney injury, alleviate renal anemia, improve mineral bone disorder, and supress the development of renal fibrosis.

Currently, no small nucleic acid-based drugs targeting this target have been approved for marketing, and there remains a need to develop such agents with improved efficacy, prolonged duration of action, enhanced target specificity, and/or better safety profiles.

### Summary

The present invention is directed to targeting the ACVR2B gene via nucleic acid therapeutics (e.g., siRNA or ASO), enabling specific and potent suppression of target genes and proteins in target organs, thereby blocking a broad spectrum of diseases triggered by aberrations in the TGF-β signaling pathway and its downstream cascades. These diseases include, but are not limited to, muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.

One aspect of the present invention provides an antisense oligonucleotide, wherein the antisense oligonucleotide comprises an oligonucleotide consisting of 12 to 30 contiguous nucleotides, and wherein the oligonucleotide comprises at least 8, at least 9, at least 10, at least 11, at least 12, at least 13, at least 14, at least 15, at least 16, at least 17, at least 18, at least 19, or at least 20 contiguous nucleotides that are at least 80%, 85%, 90%, 95% or 100% complementary to an equal-length contiguous nucleotide sequence of a target region of mRNA encoding activin type IIB receptor (ACVR2B).

In some embodiments, the target region is positions 504-554, 588-617, 648-686, 837-866, 1234-1268, 1360-1403, 1503-1532, or 1545-1574 of the nucleotide sequence set forth in SEQ ID NO: 1.

In some embodiments, the target region is positions 504-533, 510-539, 519-548, 525-554, 588-617, 648-677, 653-682, 657-686, 837-866, 1234-1263, 1239-1268, 1360-1389, 1374-1403, 1503-1532, or 1545-1574 of the nucleotide sequence set forth in SEQ ID NO: 1. In some embodiments, the target region is positions 509-528, 515-534, 524-543, 530-549, 593-612, 653-672, 658-677, 662-681, 842-861, 1239-1258, 1244-1263, 1365-1384, 1379-1398, 1508-1527, or 1550-1569 of the nucleotide sequence set forth in SEQ ID NO: 1.

In some embodiments, the oligonucleotide comprises a nucleotide sequence of at least 8, at least 9, at least 10, at least 11, at least 12, at least 13, at least 14, at least 15, at least 16, at least 17, at least 18, at least 19, or 20 contiguous nucleotides of any one of the nucleotide sequences set forth in SEQ ID NOs: 3 to 17.

In some embodiments, when measured over the entire nucleotide sequence of the antisense oligonucleotide, the antisense oligonucleotide has a nucleotide sequence that is at least 80%, at least 85%, at least 90%, at least 95% or 100% complementary to the nucleotide sequence set forth in SEQ ID NO. 1 and/or SEQ ID NO. 2.

In some embodiments, the antisense oligonucleotide comprises at least one modification selected from a modified sugar moiety, a modified nucleobase moiety, and a modified internucleoside linkage. Preferably, the antisense oligonucleotide comprises one or more of the following modifications: (i) at least one bicyclic sugar moiety, wherein the bicyclic sugar moiety preferably has a 4'-2' bridge, and the 4'-2' bridge is preferably cEt or LNA; (ii) at least one non-bicyclic modified sugar moiety, preferably selected from 2'-deoxy, 2'-F, 2'-MOE, and 2'-OMe sugar moieties; (iii) a modified nucleoside, preferably comprising 5-methylcytosine (m5C); and (iv) at least one sugar surrogate, preferably selected from POM, PNA, THP, and F-HNA.

In some embodiments, the antisense oligonucleotide comprises a gapmer. In some embodiments, the gapmer comprises: (a) a 5' region consisting of 1 to 6 linked 5' region nucleotides; (b) a central region consisting of 6 to 10 linked central region nucleotides; and (c) a 3' region consisting of 1 to 6 linked 3' region nucleotides; wherein each of the nucleotides in the 5' region and the 3' region comprises a sugar moiety selected from 2'-MOE, LNA, and cEt modified sugar moieties, and at least 6 of the nucleotides in the central region comprise a 2'-deoxy sugar moiety.

In some embodiments, the antisense oligonucleotide has a sugar motif selected from the group consisting of the following (5' to 3'): eekddddddddddkke, ekkddddddddddkke, kkkdyddddddddkkk, kkkddydddddddkkk, kkkdddyddddddkkk, kkkddddddddddkkk, and eeeeeeddddddddddeeeee; wherein e represents a 2'-MOE sugar moiety, k represents a cEt sugar moiety, d represents a 2'-deoxy sugar moiety, and y represents a 2'-OMe sugar moiety.

In some embodiments, the antisense oligonucleotide comprises at least one modified internucleoside linkage; preferably, each internucleoside linkage of the antisense oligonucleotide is a modified internucleoside linkage; preferably, the modified internucleoside linkage is selected from phosphorothioate (PS) and phosphoramidate (PN) (e.g., methanesulfonyl phosphoramidate (MsPA)), preferably PS.

In some embodiments, the antisense oligonucleotide comprises an oligonucleotide consisting of 12-30, 14-22, 14-20, 14-18, 14-20, 15-17, 15-25, or 16-20 linked nucleotides, preferably, the oligonucleotide consists of 20 contiguous nucleotides; preferably, the antisense oligonucleotide comprises, or consists of, a nucleotide sequence as set forth in any one of SEQ ID NOs: 18 to 32, or a nucleotide sequence having 1 to 3 nucleotide differences therefrom.

In some embodiments, the antisense oligonucleotide comprises a ligand; preferably, wherein the ligand comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; or the ligand comprises a ligand targeting non-hepatocyte cells, an integrin ligand, or a transferrin receptor 1 ligand, preferably the ligand is a lipophilic group. The lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

Another aspect of the present invention provides an RNAi agent for inhibiting expression of the activin type IIB receptor (ACVR2B) gene in a cell, comprising a sense strand and an antisense strand forming a duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding ACVR2B, or a nucleotide sequence having no more than 3 nucleotide differences therefrom.

In some embodiments, the target region is positions 508-534, 634-660, 655-695, 733-774, 756-782, 929-955, 1049-1089, 1141-1180, 1189-1215, 1243-1269, 1276-1321, 1377-1403, 1547-1596, 1642-1690, 1744-1773, 1916-1942, or 1949-1975 of the nucleotide sequence set forth in SEQ ID NO: 1.

In some embodiments, the target region is positions 508-534, 634-660, 655-681, 658-684, 669-695, 733-759, 748-774, 756-782, 929-955, 1049-1075, 1052-1078, 1063-1089, 1141-1167, 1154-1180, 1189-1215, 1243-1269, 1276-1302, 1280-1306, 1283-1309, 1291-1317, 1295-1321, 1377-1403, 1547-1573, 1552-1578, 1570-1596, 1642-1668, 1664-1690, 1744-1770, 1747-1773, 1916-1942, or 1949-1975 of the nucleotide sequence set forth in SEQ ID NO: 1. In some embodiments, the target region is positions 511-531, 637-657, 658-678, 661-681, 672-692, 736-756, 751-771, 759-779, 932-952, 1052-1072, 1055-1075, 1066-1086, 1144-1164, 1157-1177, 1192-1212, 1246-1266, 1279-1299, 1283-1303, 1286-1306, 1294-1314, 1298-1318, 1380-1400, 1550-1570, 1555-1575, 1573-1593, 1645-1665, 1667-1687, 1747-1767, 1750-1770, 1919-1939, or 1952-1972 of the nucleotide sequence set forth in SEQ ID NO: 1.

In some embodiments, the antisense strand comprises at least 15 contiguous nucleotides of a nucleotide sequence selected from SEQ ID NOs: 64 to 94 and nucleotide sequences having 1 to 3 nucleotide differences therefrom.

In some embodiments, the duplex region is 17 to 23 base pairs in length, preferably 18 to 21 base pairs, more preferably 19 base pairs.

In some embodiments, each of the sense strand and the antisense strand is 17 to 23 nucleotides in length, preferably 19 to 21 nucleotides.

In some embodiments, the RNAi agent comprises one or two blunt ends, preferably one blunt end.

In some embodiments, the RNAi agent comprises one or two overhangs, preferably one overhang, each overhang comprising 1 to 4 unpaired nucleotides, preferably 2 unpaired nucleotides.

In some embodiments, the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

In some embodiments, the sense strand comprises at least 15 contiguous nucleotides of any nucleotide sequence selected from SEQ ID NOs: 33 to 63 and nucleotide sequences having 1 to 3 nucleotide differences therefrom.

In some embodiments, the antisense strand has no more than 23 nucleotides and comprises a nucleotide sequence selected from SEQ ID NOs: 64 to 94; and the sense strand has no more than 21 nucleotides and comprises a nucleotide sequence from SEQ ID NOs: 33 to 63.

In some embodiments, in the RNAi agent:
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:79 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:80 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:81 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:82 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:83 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:84 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:85 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:86 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:87 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:88 or a nucleotide sequence having 1, 2, or 3 nucleotide differences therefrom;
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:89 or a nucleotide sequence having 1, 2, or 3 nucleotide differences therefrom;
the sense strand comprises or consists of a sequence set forth in SEQ ID NO:59 or a nucleotide sequence having 1, 2, or 3 nucleotide differences therefrom, and the antisense strand comprises or consists of a sequence set forth in SEQ ID NO:90 or a nucleotide sequence having 1, 2, or 3 nucleotide differences therefrom;
the sense strand comprises or consists of a sequence set forth in SEQ ID NO:60 or a nucleotide sequence having 1, 2, or 3 nucleotide differences therefrom, and the antisense strand comprises or consists of a sequence set forth in SEQ ID NO:91 or a nucleotide sequence having 1, 2, or 3 nucleotide differences therefrom;
the sense strand comprises or consists of a sequence set forth in SEQ ID NO:61 or a nucleotide sequence having 1, 2, or 3 nucleotide differences therefrom, and the antisense strand comprises or consists of a sequence set forth in SEQ ID NO:92 or a nucleotide sequence having 1, 2, or 3 nucleotide differences therefrom;
the sense strand comprises or consists of a sequence set forth in SEQ ID NO:62 or a nucleotide sequence having 1, 2, or 3 nucleotide differences therefrom, and the antisense strand comprises or consists of a sequence set forth in SEQ ID NO:93 or a nucleotide sequence having 1, 2, or 3 nucleotide differences therefrom; or
the sense strand comprises or consists of a sequence set forth in SEQ ID NO:63 or a nucleotide sequence having 1, 2, or 3 nucleotide differences therefrom, and the antisense strand comprises or consists of a sequence set forth in SEQ ID NO:94 or a nucleotide sequence having 1, 2, or 3 nucleotide differences therefrom.

In some embodiments, the RNAi agent comprises duplex 6102, 6103, 6104, 6105, 6106, 6107, 6108, 6109, 6112, 6113, 6115, 6116, 6117, 6119, 6120, 6121, 6122, 6123, 6124, 6125, 6127, 6128, 6129, 6130, 6131, 6132, 6134, 6135, 6137, 6138, or 6140.

In some embodiments, the sense strand and/or the antisense strand of the RNAi agent comprises at least one modified nucleotide, the modified nucleotide independently selected from 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

In some embodiments, in the RNAi agent, in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent, the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

In some embodiments, in the RNAi agent, in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

In some embodiments, the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

In some embodiments, the modification is selected from one of STC, ESC, Advanced ESC, ESC+, AD1-3, AD5 and GalXC.

In some embodiments, the modification is that the 5'-terminal nucleotide of the antisense strand contains a phosphate or phosphate analog modification, preferably 5'-VP.

In some embodiments, the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37. Alternatively, the RNAi agent comprises a ligand targeting non-hepatocyte cells, preferably a lipophilic group, an integrin ligand, or a transferrin receptor 1 ligand. The lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

A further aspect of the present invention provides a pharmaceutical composition comprising any ASO or RNAi agent of the present invention and a pharmaceutically acceptable carrier; preferably, the pharmaceutical composition is formulated for intravenous or subcutaneous injection.

Another aspect of the present invention provides the use of any RNAi agent of the present invention in the manufacture of a medicament for:
(i) reducing expression level of ACVR2B in a cell;
(ii) preventing or treating a disease caused by an elevated ACVR2B expression level; or
(iii) prevention or treatment of a disease selected from muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.
Yet another aspect of the present invention provides use of an agent that reduces expression of the gene encoding activin type IIB receptor (ACVR2B) in the manufacture of a medicament for the treatment of a disease or condition treatable by ACVR2B knockdown. Preferably, the agent is an antisense oligonucleotide and/or an RNAi agent. Preferably, the disease or condition is selected from muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.

Accordingly, the present invention also provides a method of reducing ACVR2B expression level in a cell, the method comprising administering to a subject in need thereof a therapeutically effective amount of any one of the antisense oligonucleotides or any one of the RNAi agents of the present invention. The present invention also provides a method for preventing or treating a disease mediated by aberrant ACVR2B expression level (e.g., caused by elevated levels thereof), the method comprising administering to a subject in need thereof a therapeutically effective amount of any one of the antisense oligonucleotides or any one of the RNAi agents of the present invention. The present invention also provides a method for preventing or treating a disease selected from muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease, the method comprising administering to a subject in need thereof a therapeutically effective amount of any one of the antisense oligonucleotides or any one of the RNAi agents of the present invention. The present invention also provides use of any one of the antisense oligonucleotides or any one of the RNAi agents of the present invention in the manufacture of a medicament for the prevention or treatment of the above-mentioned diseases. The present invention also provides any one of the antisense oligonucleotides or any one of the RNAi agents of the present invention for use in the prevention or treatment of the above-mentioned diseases.

Other aspects of the invention will be apparent from the detailed description of the specification that follows.

### Detailed Description of the Invention

### Definition

Activin A Receptor Type 2B (ACVR2B), as used herein, refers to the activin type IIB receptor protein or the gene encoding the same, also known as HTX4, ACTRIIB, or ActR-IIB, with Gene ID: 93. The NCBI accession number for the human full-length ACVR2B mRNA transcript is NM_001106.4. The NCBI accession number for the cynomolgus monkey ACVR2B mRNA transcript is XM_015445674.2.

As used herein, "oligonucleotide" refers to a nucleotide sequence that forms a chain of nucleotides via internucleoside linkages, wherein each nucleoside and internucleoside linkage may be modified or unmodified. Unless otherwise indicated, the oligonucleotide consists of 12 to 30 linked nucleotides. As used herein, "antisense oligonucleotide" or "ASO" refers to a single-stranded oligonucleotide capable of specifically binding to a target RNA (e.g., mRNA) sequence and modulating the expression of a protein (e.g., ACVR2B). As described herein, the "antisense oligonucleotide" may comprise a non-oligonucleotide moiety, such as a ligand.

"Internucleoside linkage" refers to a covalent linkage between adjacent nucleotides in an oligonucleotide. As used herein, "modified internucleoside linkage" refers to any internucleoside linkage other than a phosphodiester internucleoside linkage. "Phosphorothioate internucleoside linkage" is a modified internucleoside linkage in which one non-bridging oxygen atom of the phosphodiester internucleoside linkage is replaced by a sulfur atom.

"Deoxy region" refers to a region of 5 to 12 contiguous nucleotides, wherein at least 70% of the nucleosides are 2'-β-D-deoxynucleosides. In certain embodiments, each nucleoside is selected from 2'-β-D-deoxynucleosides, bicyclic nucleosides, and 2'-substituted nucleosides. In certain embodiments, the deoxy region supports RNase H activity. In certain embodiments, the deoxy region is the gap or internal region of a gapmer.

"Gapmer" refers to an antisense oligonucleotide comprising an internal region flanked by external regions each having one or more nucleosides, wherein the internal region comprises a plurality of nucleosides that support RNase H cleavage, and wherein the nucleosides constituting the internal region are chemically distinct from the nucleoside(s) constituting the external regions. The internal region may be referred to as the "gap," and the external regions may be referred to as the "wings." The internal region is a deoxy region. The position of the internal region or gap refers to the order of the nucleosides in the internal region and is counted from the 5' end of the internal region. Unless otherwise indicated, "gapmer" refers to a sugar motif. In certain embodiments, each nucleoside of the gap is a 2'-β-D-deoxynucleoside. In certain embodiments, the gap comprises one 2'-substituted nucleoside at position 1, 2, 3, 4, or 5 of the gap, and the remaining nucleosides of the gap are 2'-β-D-deoxynucleosides. As used herein, the term "MOE gapmer" indicates a gapmer having a gap comprising 2'-β-D-deoxynucleosides and wings comprising 2'-MOE nucleosides. As used herein, the term "mixed wing gapmer" refers to a gapmer having wings that comprise modified nucleosides containing at least two different sugar modifications. Unless otherwise indicated, a gapmer may comprise one or more modified internucleoside linkages and/or modified nucleobases, and such modifications do not necessarily follow the sugar-modified gapmer pattern.

As used herein, the term "RNAi agent" refers to a reagent comprising an RNA molecule that, when introduced into a cell, is capable of down-regulating the expression of a target gene (in this context, the ACVR2B gene) through an RNA interference mechanism. The terms "RNAi agent of the present invention," "RNAi agent described herein," or similar expressions encompass both the modified RNAi agents of the present invention, irrespective of sequence and target gene, and the RNAi agents of the present invention with specific sequences for interfering with the ACVR2B gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase.

The term "ligand" refers to a cell or tissue targeting agent that binds to a specified cell type (e.g., hepatocytes), such as a lectin, glycoprotein, lipid, or protein (e.g., an antibody). Exemplary targeting agents include thyrotropin, melanotropin, lectins, glycoproteins, surfactant protein A, mucin carbohydrates, multivalent lactose, multivalent galactose, N-acetylgalactosamine (GalNAc), multivalent (e.g., bivalent or trivalent) GalNAc, N-acetylglucosamine, multivalent mannose, multivalent fucose, glycosylated polyamino acids, multivalent galactose, transferrin, bisphosphonates, polyglutamate, polyaspartate, cholesterol, steroids, bile acids, folate, vitamin B12, biotin, RGD peptides, and RGD peptide mimetics. In preferred embodiments, the ligand is a carbohydrate, such as a monosaccharide, disaccharide, trisaccharide, tetrasaccharide, or polysaccharide. For example, the ligand may be a derivative comprising GalNAc. In preferred embodiments, the ligand is a derivative comprising one or more N-acetylgalactosamine moieties attached via a bivalent or trivalent branched linker.

The term "therapeutically effective amount" refers to an amount of an RNAi agent or composition of the present invention that is effective to produce a desired therapeutic effect in at least a subpopulation of cells in an animal at a reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting an ASO or RNAi agent from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

### Antisense Oligonucleotides for Inhibiting ACVR2B Gene Expression

A first aspect of the present invention provides an antisense oligonucleotide, comprising an oligonucleotide consisting of 12 to 30 contiguous nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides that are at least 80%, 85%, 90%, 95%, or 100% complementary to an equal-length contiguous nucleotide sequence of a target region of mRNA encoding activin type IIB receptor (ACVR2B).

In some embodiments, said target region is nucleotides 504-554, 588-617, 648-686, 837-866, 1234-1268, 1360-1403, 1503-1532, or 1545-1574 of the nucleotide sequence set forth in SEQ ID NO: 1. In some embodiments, said target region is nucleotides 5504-533, 510-539, 519-548, 525-554, 588-617, 648-677, 653-682, 657-686, 837-866, 1234-1263, 1239-1268, 1360-1389, 1374-1403, 1503-1532, or 1545-1574 of the nucleotide sequence set forth in SEQ ID NO: 1. In preferred embodiments, said target region is nucleotides 509-528, 515-534, 524-543, 530-549, 593-612, 653-672, 658-677, 662-681, 842-861, 1239-1258, 1244-1263, 1365-1384, 1379-1398, 1508-1527, or 1550-1569 of the nucleotide sequence set forth in SEQ ID NO: 1.

Therefore, in some embodiments, the present invention provides an antisense oligonucleotide, comprising an oligonucleotide consisting of 12 to 30 contiguous nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides that are at least 80%, 85%, 90%, 95%, or 100% complementary to an equal-length contiguous nucleotide sequence within nucleotides 504-554, 588-617, 648-686, 837-866, 1234-1268, 1360-1403, 1503-1532, or 1545-1574 of the nucleotide sequence set forth in SEQ ID NO: 1.

In some embodiments, the present invention provides an antisense oligonucleotide, comprising an oligonucleotide consisting of 12 to 30 contiguous nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides that are at least 80%, 85%, 90%, 95%, or 100% complementary to an equal-length contiguous nucleotide sequence within nucleotides504-533, 510-539, 519-548, 525-554, 588-617, 648-677, 653-682, 657-686, 837-866, 1234-1263, 1239-1268, 1360-1389, 1374-1403, 1503-1532, or 1545-1574 of the nucleotide sequence set forth in SEQ ID NO: 1.

In some embodiments, the present invention provides an antisense oligonucleotide, comprising an oligonucleotide consisting of 12 to 30 contiguous nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides that are at least 80%, 85%, 90%, 95%, or 100% complementary to an equal-length contiguous nucleotide sequence within nucleotides 509-528, 515-534, 524-543, 530-549, 593-612, 653-672, 658-677, 662-681, 842-861, 1239-1258, 1244-1263, 1365-1384, 1379-1398, 1508-1527, or 1550-1569 of the nucleotide sequence set forth in SEQ ID NO: 1.

In some embodiments, the oligonucleotide comprises at least 8, at least 9, at least 10, at least 11, at least 12, at least 13, at least 14, at least 15, at least 16, at least 17, at least 18, at least 19, or at least 20 contiguous nucleotides of the nucleotide sequence of any one of SEQ ID NOs: 3 to 17.

In some embodiments, the oligonucleotide consists of 12-30, 14-22, 14-20, 14-18, 14-20, 15-17, 15-25 or 16-20 linked nucleotides and has a nucleotide sequence comprising at least 8, at least 9, at least 10, at least 11, at least 12, at least 13, at least 14, at least 15, at least 16, at least 17, at least 18, at least 19, or at least 20 contiguous nucleotides of the nucleotide sequence of any one of SEQ ID NOs: 3 to 17, and wherein when measured over the entire nucleotide sequence of the antisense oligonucleotide, the antisense oligonucleotide has a nucleotide sequence that is at least 80%, at least 85%, at least 90%, at least 95% or 100% (preferably 100%) complementary to the nucleotide sequence of the messenger RNA (mRNA) of human and/or cynomolgus monkey activin type IIB receptor (ACVR2B) (NCBI accession numbers NM_001106.4, SEQ ID NO. 1 and XM_015445674.2, SEQ ID NO. 2, respectively).

In some embodiments, the provided antisense oligonucleotides do not comprise modified nucleotides, such as modified sugar moieties, modified nucleobase moieties, or modified internucleoside linkages. In such embodiments, for example, the antisense oligonucleotide provided by the present invention may comprise the nucleotide sequence set forth in any one of SEQ ID NOs: 3, 4, 5, 6, 7, 8, 9, 10, 11, 12, 13, 14, 15, 16, or 17, or a nucleotide sequence having 1, 2, or 3 nucleotide differences therefrom.

In some embodiments, the provided antisense oligonucleotides comprise modified nucleotides, such as modified sugar moieties and/or modified nucleobase moieties and/or modified internucleoside linkages. In preferred embodiments, the provided antisense oligonucleotide comprises modified nucleotides, including modified sugar moieties, modified nucleobase moieties, and modified internucleoside linkages.

In some embodiments, the antisense oligonucleotide comprises an oligonucleotide consisting of 12-30, 14-22, 14-20, 14-18, 14-20, 15-17, 15-25, or 16-20 linked nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides of the nucleotide sequence of any one of SEQ ID NOs: 3 to 17, wherein the antisense oligonucleotide comprises at least one modification selected from the group consisting of a modified sugar moiety, a modified nucleobase moiety, and a modified internucleoside linkage.

In some embodiments, every nucleoside in the oligonucleotide comprised in the antisense oligonucleotide is modified, said modification comprising a modified sugar moiety and a modified internucleoside linkage.

In some embodiments, every nucleoside in the oligonucleotide comprised in the antisense oligonucleotide is modified, said modification comprising a modified sugar moiety, a modified nucleobase moiety, and a modified internucleoside linkage.

In some embodiments, the modified sugar moiety may be at least one bicyclic sugar moiety, at least one non-bicyclic modified sugar moiety, and/or at least one sugar surrogate.

In some embodiments, the antisense oligonucleotide comprises at least one bicyclic sugar moiety. In some embodiments, a substituent bridges two atoms of a furanosyl ring to form a second ring, thereby generating a bicyclic sugar moiety. In certain such embodiments, the bicyclic sugar moiety comprises a bridge between the 4' and 2' furanosyl ring atoms. Examples of such 4' to 2' bridging sugar substituents include, but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt"), 4'-CH₂-O-CH₂-2', 4'-CH₂-N(R)-2', 4'-CH(CH₂OCH₃)-O-2' ("constrained MOE" or "cMOE") and analogs thereof (see, e.g., Seth et al., U.S. 7,399,845; Bhat et al., U.S. 7,569,686; Swayze et al., U.S. 7,741,457; and Swayze et al., U.S. 8,022,193), 4'-C(CH₃)(CH₃)-O-2' and analogs thereof (see, e.g., Seth et al., U.S. 8,278,283), 4'-CH₂-N(OCH₃)-2' and analogs thereof (see, e.g., Prakash et al., U.S. 8,278,425), 4'-CH₂-O-N(CH₃)-2' (see, e.g., Allerson et al., U.S. 7,696,345 and Allerson et al., U.S. 8,124,745), 4'-CH₂-C(H)(CH₃)-2' (see, e.g., Zhou et al., J. Org. Chem., 2009, 74, 118-134), 4'-CH₂-C(=CH₂)-2' and analogs thereof (see, e.g., Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is independently H, a protecting group, or C₁-C₁₂ alkyl (see, e.g., Imanishi et al., U.S. 7,427,672). In certain embodiments, bicyclic sugar moieties and nucleosides incorporating such bicyclic sugar moieties are further defined by isomeric configuration. For example, LNA nucleosides (as described herein) may be in the α-L configuration or in the β-D configuration. In preferred embodiments, the bicyclic sugar moiety has a 4'-2' bridge, which is preferably cEt or LNA.

In some embodiments, the antisense oligonucleotide comprises at least one non-bicyclic modified sugar moiety, and such non-bridging substituents may be located at any position of the furanosyl ring, including, but not limited to, substituents at the 2'-position, the 4'-position, and/or the 5'-position. In certain embodiments, one or more non-bridging substituents of the non-bicyclic modified sugar moiety are branched. Examples of 2'-substituents suitable for non-bicyclic modified sugar moieties include, but are not limited to: 2'-F, 2'-OCH₃ ("OMe" or "O-methyl"), and 2'-O(CH₂)₂OCH₃ ("MOE" or "O-methoxyethyl"). In certain embodiments, the 2'-substituent is selected from: halo, allyl, amino, azido, SH, CN, OCN, CF₃, OCF₃, O-C₁-C₁₀ alkoxy, substituted O-C₁-C₁₀ alkoxy, O-C₁-C₁₀ alkyl, substituted O-C₁-C₁₀ alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylene-O-alkyl, alkynyl, alkaryl, aralkyl, O-alkaryl, O-aralkyl, O(CH₂)₂SCH₃, O(CH₂)₂ON(Rₘ)(Rₙ), or OCH₂C(=O)-N(Rₘ)(Rₙ), wherein each Rₘ and Rₙ is independently H, an amino protecting group, or substituted or unsubstituted C₁-C₁₀ alkyl, as well as the 2'-substituents set forth in Cook et al., U.S. 6,531,584; Cook et al., U.S. 5,859,221; and Cook et al., U.S. 6,005,087. Certain embodiments of these 2'-substituents may be further substituted with one or more substituents independently selected from: hydroxyl, amino, alkoxy, carboxyl, benzyl, phenyl, nitro (NO₂), thiol, thioalkoxy, thioalkyl, halogen, alkyl, aryl, alkenyl, and alkynyl. Examples of 4'-substituents suitable for non-bicyclic modified sugar moieties include, but are not limited to, alkoxy (e.g., methoxy), alkyl, and those groups set forth in Manoharan et al., WO 2015/106128. Examples of 5'-substituents suitable for non-bicyclic modified sugar moieties include, but are not limited to: 5'-methyl (R or S), 5'-vinyl, and 5'-methoxy. In certain embodiments, the non-bicyclic modified sugar moiety comprises more than one non-bridging sugar substituent, such as a 2'-F-5'-methyl sugar moiety and modified sugar moieties and modified nucleosides as set forth in Migawa et al., WO 2008/101157 and Rajeev et al., US 2013/0203836.

In certain embodiments, the 2'-substituted non-bicyclic modified nucleoside comprises a sugar moiety comprising a non-bridging 2'-substituent selected from the group consisting of: F, NH₂, N₃, OCF₃, OCH₃, O(CH₂)₃NH₂, CH₂CH = CH₂, OCH₂CH = CH₂, OCH₂CH₂OCH₃, O(CH₂)₂SCH₃, O(CH₂)₂ON(Rₘ)(Rₙ), O(CH₂)₂O(CH₂)₂N(CH₃)₂, and N-substituted acetamide (OCH₂C(=O)-N(Rₘ)(Rₙ)), wherein each Rₘ and Rₙ is independently H, an amino protecting group, or substituted or unsubstituted C₁-C₁₀ alkyl.

In certain embodiments, the 2'-substituted non-bicyclic modified nucleoside comprises a sugar moiety comprising a non-bridging 2'-substituent selected from the group consisting of: F, OCF₃, OCH₃, OC H₂CH₂OCH₃, O(CH₂)₂SCH₃, O(CH₂)₂ON(CH₃)₂, O(CH₂)₂O(CH₂)₂N(CH₃)₂, and OCH₂C(=O)-N(H)CH₃("NMA"). In certain embodiments, the 2'-substituted non-bicyclic modified nucleoside comprises a sugar moiety comprising a non-bridging 2'-substituent selected from the group consisting of: F (2'-F), OCH₃ (2'-OMe), and OCH₂CH₂OCH₃ (2'-MOE).

In certain embodiments, the modified furanosyl sugar moiety and the nucleoside incorporating such a modified furanosyl sugar moiety are further defined by isomeric configuration. For example, a 2'-deoxyfuranosyl sugar moiety may be in any of seven isomeric configurations other than the naturally occurring β-D-deoxyribosyl configuration. Such modified sugar moieties are set forth in, for example, WO 2019/157531, which is incorporated herein by reference. A 2'-modified sugar moiety has an additional stereocenter at the 2'-position relative to the 2'-deoxyfuranosyl sugar moiety; therefore, such sugar moieties have a total of sixteen possible isomeric configurations. Unless otherwise specified, the 2'-modified sugar moieties described herein are in the β-D-ribosyl isomeric configuration.

In some embodiments, the antisense oligonucleotide comprises at least one sugar surrogate. In certain such embodiments, the oxygen atom of the sugar moiety is replaced by, for example, a sulfur, carbon, or nitrogen atom. In certain such embodiments, such modified sugar moieties also comprise bridging and/or non-bridging substituents as set forth herein. For example, certain sugar surrogates comprise a 4'-sulfur atom and substituents at the 2'-position (see, e.g., Bhat et al., U.S. 7,875,733 and Bhat et al., U.S. 7,939,677) and/or the 5'-position.

In certain embodiments, the sugar surrogate comprises a ring having other than five atoms. For example, in certain embodiments, the sugar surrogate comprises a six-membered tetrahydropyran ("THP"). Such tetrahydropyrans may be further modified or substituted. Nucleosides comprising such modified tetrahydropyrans include, but are not limited to, hexitol nucleic acid ("HNA"), anitol nucleic acid ("ANA"), mannitol nucleic acid ("MNA") (see, e.g., Leumann, C. J. Bioorg. & Med. Chem. 2002, 10, 841-854), and fluoro HNA (F-HNA, also referred to as F-THP or 3'-fluoro tetrahydropyran).

In certain embodiments, the sugar surrogate comprises a ring having more than five atoms and more than one heteroatom. For example, nucleosides comprising a morpholino sugar moiety and their use in oligonucleotides have been reported. In certain embodiments, the sugar surrogate comprises an acyclic moiety. Examples of nucleosides and oligonucleotides comprising such acyclic sugar surrogates include, but are not limited to: peptide nucleic acid ("PNA"), acyclic butyl nucleic acid (see, e.g., Kumar et al., Org. Biomol. Chem., 2013, 11, 5853-5865), and the nucleosides and oligonucleotides set forth in Manoharan et al., WO 2011/133876. By introducing a modified nucleobase having a cationic lipid or its equivalent covalently linked thereto, PNA can be rendered highly permeable to mammalian cell membranes (see, e.g., WO 2019/022434 and WO 2009/113828). In preferred embodiments, the sugar surrogate is selected from POM, PNA, THP, and F-HNA.

In some embodiments, the antisense oligonucleotide comprises one or more nucleosides comprising an unmodified nucleobase. In certain embodiments, the antisense oligonucleotide comprises one or more nucleosides comprising a modified nucleobase. In certain embodiments, the antisense oligonucleotide comprises one or more nucleosides that do not comprise a nucleobase, referred to as abasic nucleosides.

In certain embodiments, the modified nucleobase is selected from: 5-substituted pyrimidines (e.g., 5-methylcytosine, m5C), 6-azapyrimidines, alkyl- or alkynyl-substituted pyrimidines, alkyl-substituted purines, and N-2, N-6, and O-6 substituted purines. In certain embodiments, the modified nucleobase is selected from: 2-aminopropyladenine, 5-hydroxymethylcytosine, xanthine, hypoxanthine, 2-aminoadenine, 6-N-methylguanine, 6-N-methyladenine, 2-propyladenine, 2-thiouracil, 2-thiothymine, and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil; 8-halo-, 8-amino-, 8-thiol-, 8-thioalkyl-, 8-hydroxy-, 8-aza,- and other 8-substituted purines; 5-halo-, particularly 5-bromo-, 5-trifluoromethyl-, 5-halouracil, and 5-halocytosine; 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 5-methylcytosine, 4-N-benzoylcytosine, 4-N-benzoyluracil, 5-methyl-4-N-benzoylcytosine, 5-methyl-4-N-benzoyluracil, universal bases, hydrophobic bases, promiscuous bases, size-expanded bases, and fluorinated bases. Other modified nucleobases include tricyclic pyrimidines, such as 1,3-diazaphenoxazine-2-one, 1,3-diazaphenothiazine-2-one, and 9-(2-aminoethoxy)-1,3-diazaphenoxazine-2-one (G-clamp). Modified nucleobases may also include those in which the purine or pyrimidine base is replaced by other heterocycles, such as 7-deaza-adenine, 7-deazaguanosine, 2-aminopyridine, and 2-pyridone. In preferred embodiments, the antisense oligonucleotide comprises one or more 5-methylcytosines (m5C).

In some embodiments, the modified nucleobase may be a nucleobase having a cationic lipid covalently linked thereto. By introducing a modified nucleobase having a cationic lipid or an equivalent thereof covalently linked thereto, the antisense oligonucleotide can be rendered highly permeable to mammalian cell membranes. Suitable cationic lipid-linked modifications covalently attached thereto may be those described in WO 2019022434A1, which is incorporated herein by reference in its entirety.

In some embodiments, the antisense oligonucleotide comprises at least one modified internucleoside linkage. In preferred embodiments, every internucleoside linkage of the antisense oligonucleotide is a modified internucleoside linkage. In certain embodiments, any internucleoside linkage may be used to link the nucleosides of the antisense oligonucleotide together. Two main classes of internucleoside linking groups are defined by the presence or absence of a phosphorus atom. Representative phosphorus-containing internucleoside linkages include, but are not limited to, phosphodiester, which contains a phosphodiester bond ("P(O₂)=O") (also referred to as an unmodified linkage or naturally occurring linkage); phosphotriester; methylphosphonate; phosphoramidate (PN); phosphorothioate ("P(O₂)=S", PS); and phosphorodithioate ("HS-P=S"). Phosphoramidate (PN) may have the structural formula - N(R)P(=X)(OH)O-, -OP(=X)(OH)N(R)-, -O-P(NR)(=X)O-, - N(SO2R)P(=X)(OH)O-, - OP(=X)(OH)N(SO₂R)-, or -O-P(NSO₂R)(=X)O- ("MsPA"), wherein X is O or S, R may be optionally substituted alkyl, aryl, heteroaryl, or heterocycloalkyl; or NR may be an optionally substituted cyclic guanidinyl moiety, an optionally substituted triazolyl group, or a Tmg group. Further PN structures can be found in WO 2023220744, WO 2019/032612, and WO 2021/030778, which are incorporated herein by reference in their entirety.

Representative phosphorus-free internucleoside linking groups include, but are not limited to, methylenemethylimino (-CH₂-N(CH₃)-O-CH₂-), thiodiester, thiocarbamate (-O-C( = O)(NH)-S-); siloxane (-O-SiH₂-O-); and N,N'-dimethylhydrazine (-CH₂-N(CH₃)-N(CH₃)-). Modified internucleoside linkages can be used to alter, and typically increase, the nuclease resistance of oligonucleotides compared to naturally occurring phosphodiester internucleoside linkages. In certain embodiments, internucleoside linkages having a chiral atom can be prepared as racemic mixtures or as separated enantiomers. Methods for preparing phosphorus-containing and phosphorus-free internucleoside linkages are well known to those skilled in the art.

Representative internucleoside linkages having a chiral center include, but are not limited to, alkylphosphonates and phosphorothioates. Antisense oligonucleotides comprising internucleoside linkages having a chiral center may be prepared as a population of antisense oligonucleotides comprising stereorandom internucleoside linkages, or as a population of antisense oligonucleotides comprising phosphorothioate internucleoside linkages in a specific stereochemical configuration. In certain embodiments, a population of antisense oligonucleotides comprises phosphorothioate internucleoside linkages, wherein all of said phosphorothioate internucleoside linkages are stereorandom. Such antisense oligonucleotides can be generated using synthetic methods that randomly select the stereochemical configuration of each phosphorothioate internucleoside linkage. Nevertheless, as is well understood by those skilled in the art, each individual phosphorothioate linkage of each individual oligonucleotide molecule has a defined stereochemical configuration. In certain embodiments, the population of antisense oligonucleotides is enriched for antisense oligonucleotides comprising one or more specific phosphorothioate internucleoside linkages in a specific, independently selected stereochemical configuration. In certain embodiments, at least 65% of the molecules in the population have the specific phosphorothioate internucleoside linkage in a specific configuration. In certain embodiments, at least 70% of the molecules in the population have the specific phosphorothioate internucleoside linkage in a specific configuration. In certain embodiments, at least 80% of the molecules in the population have the specific phosphorothioate internucleoside linkage in a specific configuration. In certain embodiments, at least 90% of the molecules in the population have the specific phosphorothioate internucleoside linkage in a specific configuration. In certain embodiments, at least 99% of the molecules in the population have the specific phosphorothioate internucleoside linkage in a specific configuration. Such chirally enriched populations of antisense oligonucleotides can be generated using synthetic methods known in the art, such as those set forth in Oka et al., JACS 125, 8307 (2003); Wan et al., Nuc. Acid. Res. 42, 13456 (2014); and WO 2017/015555. In certain embodiments, the population of antisense oligonucleotides is enriched for antisense oligonucleotides having at least one indicated phosphorothioate linkage in the (Sp) configuration. In certain embodiments, the population of antisense oligonucleotides is enriched for antisense oligonucleotides having at least one phosphorothioate linkage in the (Rp) configuration.

In some embodiments, the antisense oligonucleotide comprises a gapmer. Said gapmer is defined by two external regions or "wings" and a central or internal region or "gap." The three regions of the gapmer motif (5'-wing, gap, and 3'-wing) form a contiguous sequence of nucleosides, wherein at least some sugar moieties of the nucleosides of each wing are different from at least some sugar moieties of the nucleosides of the gap. Specifically, at least the sugar moiety of the nucleoside in each wing that is closest to the gap (the 3'-most nucleoside of the 5'-wing and the 5'-most nucleoside of the 3'-wing) is different from the sugar moiety of the adjacent gap nucleoside, thereby defining the boundary between the wing and the gap (i.e., the wing/gap junction). In certain embodiments, the sugar moieties within the gap are identical to each other. In certain embodiments, the gap comprises one or more nucleosides having a sugar moiety that is different from the sugar moiety of one or more other nucleosides of the gap. In certain embodiments, the sugar motifs of the two wings are identical to each other (symmetric gapmer). In certain embodiments, the sugar motif of the 5'-wing is different from the sugar motif of the 3'-wing (asymmetric gapmer).

In some embodiments, the gapmer comprises: (a) a 5' region consisting of 1-6 linked 5' region nucleosides; (b) a central region consisting of 6-10 linked central region nucleosides; and (c) a 3' region consisting of 1-6 linked 3' region nucleosides; wherein each of the nucleosides in the 5' region and the 3' region comprises a sugar moiety selected from 2'-MOE, LNA, and cEt modifications, and at least 6 of the nucleosides in the central region comprise a 2'-deoxy sugar moiety.

In some embodiments, the gapmer has a sugar motif (5' to 3') selected from: eekddddddddddkke, ekkddddddddddkke, kkkdyddddddddkkk, kkkddydddddddkkk, kkkdddyddddddkkk, kkkddddddddddkkk, or eeeeeddddddddddeeeee; wherein e represents a 2'-MOE sugar moiety, k represents a cEt sugar moiety, d represents a 2'-deoxy sugar moiety, and y represents a 2'-OMe sugar moiety.

In preferred embodiments, the gapmer has the sugar motif eeeeeeddddddddddeeeee (5' to 3'), wherein e represents a 2'-MOE sugar moiety and d represents a 2'-deoxy sugar moiety.

In preferred embodiments, the internucleoside linkages of the gapmer are PS. In such embodiments, the gapmer comprises or consists of a nucleotide sequence as set forth in any one of SEQ ID NOs: 18 to 32, or a nucleotide sequence having 1, 2, or 3 nucleotide differences therefrom. In one embodiment, the gapmer comprises or consists of a nucleotide sequence as set forth in SEQ ID NO: 18, or a nucleotide sequence having 1, 2, or 3 nucleotide differences therefrom. In one embodiment, the gapmer comprises or consists of a nucleotide sequence as set forth in SEQ ID NO: 19, or a nucleotide sequence having 1, 2, or 3 nucleotide differences therefrom. In one embodiment, the gapmer comprises or consists of a nucleotide sequence as set forth in SEQ ID NO: 20, or a nucleotide sequence having 1, 2, or 3 nucleotide differences therefrom. In one embodiment, the gapmer comprises or consists of a nucleotide sequence as set forth in SEQ ID NO: 21, or a nucleotide sequence having 1, 2, or 3 nucleotide differences therefrom. In one embodiment, the gapmer comprises or consists of a nucleotide sequence as set forth in SEQ ID NO: 22, or a nucleotide sequence having 1, 2, or 3 nucleotide differences therefrom. In one embodiment, the gapmer comprises or consists of a nucleotide sequence as set forth in SEQ ID NO: 23, or a nucleotide sequence having 1, 2, or 3 nucleotide differences therefrom. In one embodiment, the gapmer comprises or consists of a nucleotide sequence as set forth in SEQ ID NO: 24, or a nucleotide sequence having 1, 2, or 3 nucleotide differences therefrom. In one embodiment, the gapmer comprises or consists of a nucleotide sequence as set forth in SEQ ID NO: 25, or a nucleotide sequence having 1, 2, or 3 nucleotide differences therefrom. In one embodiment, the gapmer comprises or consists of a nucleotide sequence as set forth in SEQ ID NO: 26, or a nucleotide sequence having 1, 2, or 3 nucleotide differences therefrom. In one embodiment, the gapmer comprises or consists of a nucleotide sequence as set forth in SEQ ID NO: 27, or a nucleotide sequence having 1, 2, or 3 nucleotide differences therefrom. In one embodiment, the gapmer comprises or consists of a nucleotide sequence as set forth in SEQ ID NO: 28, or a nucleotide sequence having 1, 2, or 3 nucleotide differences therefrom. In one embodiment, the gapmer comprises or consists of a nucleotide sequence as set forth in SEQ ID NO: 29, or a nucleotide sequence having 1, 2, or 3 nucleotide differences therefrom. In one embodiment, the gapmer comprises or consists of a nucleotide sequence as set forth in SEQ ID NO: 30, or a nucleotide sequence having 1, 2, or 3 nucleotide differences therefrom. In one embodiment, the gapmer comprises or consists of a nucleotide sequence as set forth in SEQ ID NO: 31, or a nucleotide sequence having 1, 2, or 3 nucleotide differences therefrom. In one embodiment, the gapmer comprises or consists of a nucleotide sequence as set forth in SEQ ID NO: 32, or a nucleotide sequence having 1, 2, or 3 nucleotide differences therefrom.

### RNAi Agents For Inhibiting ACVR2B Gene Expression

One aspect of the present invention provides an RNAi agent for inhibiting ACVR2B gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding ACVR2B, or a nucleotide sequence having no more than 3 nucleotide differences therefrom.

In some embodiments, the target region is positions 508-534, 634-660, 655-695, 733-774, 756-782, 929-955, 1049-1089, 1141-1180, 1189-1215, 1243-1269, 1276-1321, 1377-1403, 1547-1596, 1642-1690, 1744-1773, 1916-1942, or 1949-1975 of the nucleotide sequence set forth in SEQ ID NO: 1. In some embodiments, the target region is positions 508-534, 634-660, 655-681, 658-684, 669-695, 733-759, 748-774, 756-782, 929-955, 1049-1075, 1052-1078, 1063-1089, 1141-1167, 1154-1180, 1189-1215, 1243-1269, 1276-1302, 1280-1306, 1283-1309, 1291-1317, 1295-1321, 1377-1403, 1547-1573, 1552-1578, 1570-1596, 1642-1668, 1664-1690, 1744-1770, 1747-1773, 1916-1942, or 1949-1975 of the nucleotide sequence set forth in SEQ ID NO: 1. In some embodiments, the target region is positions 511-531, 637-657, 658-678, 661-681, 672-692, 736-756, 751-771, 759-779, 932-952, 1052-1072, 1055-1075, 1066-1086, 1144-1164, 1157-1177, 1192-1212, 1246-1266, 1279-1299, 1283-1303, 1286-1306, 1294-1314, 1298-1318, 1380-1400, 1550-1570, 1555-1575, 1573-1593, 1645-1665, 1667-1687, 1747-1767, 1750-1770, 1919-1939, or 1952-1972 of the nucleotide sequence set forth in SEQ ID NO: 1.

Therefore, in some embodiments, the present invention provides an RNAi agent for inhibiting ACVR2B gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 508-534, 634-660, 655-695, 733-774, 756-782, 929-955, 1049-1089, 1141-1180, 1189-1215, 1243-1269, 1276-1321, 1377-1403, 1547-1596, 1642-1690, 1744-1773, 1916-1942, or 1949-1975 of the nucleotide sequence set forth in SEQ ID NO: 1, or a nucleotide sequence having no more than 3 nucleotide differences therefrom.

In preferable embodiments, the present invention provides an RNAi agent for inhibiting ACVR2B gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 508-534, 634-660, 655-681, 658-684, 669-695, 733-759, 748-774, 756-782, 929-955, 1049-1075, 1052-1078, 1063-1089, 1141-1167, 1154-1180, 1189-1215, 1243-1269, 1276-1302, 1280-1306, 1283-1309, 1291-1317, 1295-1321, 1377-1403, 1547-1573, 1552-1578, 1570-1596, 1642-1668, 1664-1690, 1744-1770, 1747-1773, 1916-1942, or 1949-1975 of the nucleotide sequence set forth in SEQ ID NO: 1, or a nucleotide sequence having no more than 3 nucleotide differences therefrom.

In preferable embodiments, the present invention provides an RNAi agent for inhibiting ACVR2B gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 511-531, 637-657, 658-678, 661-681, 672-692, 736-756, 751-771, 759-779, 932-952, 1052-1072, 1055-1075, 1066-1086, 1144-1164, 1157-1177, 1192-1212, 1246-1266, 1279-1299, 1283-1303, 1286-1306, 1294-1314, 1298-1318, 1380-1400, 1550-1570, 1555-1575, 1573-1593, 1645-1665, 1667-1687, 1747-1767, 1750-1770, 1919-1939, or 1952-1972 of the nucleotide sequence set forth in SEQ ID NO: 1, or a nucleotide sequence having no more than 3 nucleotide differences therefrom.

In some embodiments, the antisense strand comprises at least 15 (e.g., 15, 16, 17, 18, 19, 20 or 21) contiguous nucleotides of a nucleotide sequence selected from SEQ ID NOs: 64 to 94 and nucleotide sequences having 1, 2, or 3 nucleotide differences each therefrom.

In some embodiments, the present invention provides an RNAi agent for inhibiting ACVR2B gene expression, comprising a sense strand and an antisense strand that form a complementary duplex region, wherein the antisense strand comprises at least 15 (e.g., 15, 16, 17, 18, 19, 20, or 21) contiguous nucleotides selected from any one of the nucleotide sequences of SEQ ID NOs: 64 to 94.

In some embodiments, the present invention provides an RNAi agent for inhibiting ACVR2B gene expression, comprising a sense strand and an antisense strand that form a complementary duplex region, wherein the antisense strand comprises at least 15 (e.g., 15, 16, 17, 18, 19, 20, or 21) contiguous nucleotides selected from a nucleotide sequence that differs from any one of the nucleotide sequences of SEQ ID NOs: 64 to 94 by 1, 2, or 3 nucleotides.

In the present invention, when referring to 1, 2, or 3 nucleotide differences, the 1, 2, or 3 nucleotide differences may be on the sense strand and/or antisense strand outside the duplex region. In some embodiments, the 1, 2, or 3 nucleotide differences are on the sense strand and/or antisense strand within the duplex region. In some embodiments, a portion of the 1, 2, or 3 nucleotide differences are on the sense strand and/or antisense strand within the duplex region, and another portion are on the sense strand and/or antisense strand outside the duplex region. For the antisense strand, in some embodiments, the 1, 2, or 3 nucleotide differences are located at the extreme 3' end or extreme 5' end of the antisense strand. In some embodiments, the 1, 2, or 3 nucleotide differences are located between the extreme 3' end and extreme 5' end of the antisense strand. In some embodiments, 1 or 2 of the 1, 2, or 3 nucleotide differences are located at the extreme 3' end or extreme 5' end of the antisense strand, and the other 1 or 2 are located between the extreme 3' end and extreme 5' end of the antisense strand. For the sense strand, in some embodiments, the 1, 2, or 3 nucleotide differences are located at the extreme 3' end or extreme 5' end of the sense strand. In some embodiments, the 1, 2, or 3 nucleotide differences are located between the extreme 3' end and extreme 5' end of the sense strand. In some embodiments, 1 or 2 of the 1, 2, or 3 nucleotide differences are located at the extreme 3' end or extreme 5' end of the sense strand, and the other 1 or 2 are located between the extreme 3' end and extreme 5' end of the sense strand.

In some embodiments, the present invention provides an RNAi agent for inhibiting ACVR2B gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 1 nucleotide difference from any nucleotide sequence selected from SEQ ID NOs: 64 to 94, the 1 nucleotide difference being located at the extreme 3' end of the antisense strand. In some embodiments, the present invention provides an RNAi agent for inhibiting ACVR2B gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 2 nucleotide differences from any nucleotide sequence selected from SEQ ID NOs: 64 to 94, the 2 nucleotide differences being located consecutively at the extreme 3' end of the antisense strand.

In some embodiments, the present invention provides an RNAi agent for inhibiting ACVR2B gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of any nucleotide sequence selected from SEQ ID NOs: 64 to 94.

In some embodiments, the present invention provides an RNAi agent for inhibiting ACVR2B gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 64 to 94.

One aspect of the present invention provides an RNAi agent for inhibiting ACVR2B gene expression, comprising an antisense strand, wherein the antisense strand comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom.

In some embodiments, the present invention provides an RNAi agent for inhibiting ACVR2B gene expression, comprising an antisense strand, wherein the antisense strand comprises at least 15 (e.g., 15, 16, 17, 18, 19, 20, or 21) contiguous nucleotides of any nucleotide sequence selected from SEQ ID NOs: 64 to 94.

In some embodiments, the present invention provides an RNAi agent for inhibiting ACVR2B gene expression, comprising an antisense strand, wherein the antisense strand comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 64 to 94.

In some embodiments, the length of the duplex region is about 17 to about 23 base pairs. For example, suitable lengths for the duplex region are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to 23 base pairs, about 19 to 22 base pairs, about 19 to 21 base pairs, about 20 to 23 base pairs, about 20 to 22 base pairs, or about 21 to 23 base pairs. In certain embodiments, the length of the duplex region is about 18 to about 21 base pairs. In other embodiments, the length of the duplex region is about 19 base pairs.

Accordingly, in some embodiments of the present invention, an RNAi agent for inhibiting ACVR2B gene expression is provided, comprising a sense strand and an antisense strand that form a complementary duplex region, the length of the duplex region being about 17 to about 23 base pairs, and wherein the antisense strand is no more than 23 nucleotides in length and comprises at least 15 contiguous nucleotides of a nucleotide sequence selected from SEQ ID NOs: 64 to 94 and nucleotide sequences having 1 to 3 nucleotide differences therefrom.

In some embodiments of the present invention, an RNAi agent for inhibiting ACVR2B gene expression is provided, comprising a sense strand and an antisense strand that form a complementary duplex region, the length of the duplex region being about 18 to about 21 base pairs, and wherein the antisense strand is no more than 23 nucleotides in length and comprises at least 15 contiguous nucleotides of a nucleotide sequence selected from SEQ ID NOs: 64 to 94 and nucleotide sequences having 1 to 3 nucleotide differences therefrom.

In some embodiments of the present invention, an RNAi agent for inhibiting ACVR2B gene expression is provided, comprising a sense strand and an antisense strand that form a complementary duplex region, the length of the duplex region being about 19 base pairs, and wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom.

In some embodiments, the sense and antisense strands of the RNAi agent of the invention each independently have a length of about 17 to about 23 nucleotides, e.g., about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense and antisense strands each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting ACVR2B gene expression comprises two nucleotide overhangs. For example, in one embodiment, the RNAi agent for inhibiting ACVR2B gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting ACVR2B gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting ACVR2B gene expression is blunt-ended and comprises (i) a sense strand and an antisense strand each having a length of 21 nucleotides, and (ii) a duplex region having a length of 21 base pairs. In another such embodiment, the RNAi agent for inhibiting ACVR2B gene expression is blunt-ended and comprises (i) a sense strand and an antisense strand each having a length of 23 nucleotides, and (ii) a duplex region having a length of 23 base pairs. In another such embodiment, the RNAi agent for inhibiting ACVR2B gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting ACVR2B gene expression comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting ACVR2B gene expression comprises nucleotide overhangs at both 5'-end and 3'-end of the antisense strand. In another embodiment, the RNAi agent for inhibiting ACVR2B gene expression comprises nucleotide overhangs at both 5'-end and 3'-end of the sense strand. In some embodiments, the RNAi agent for inhibiting ACVR2B gene expression comprises nucleotide overhangs at the 5'-end of the sense strand and the 5'-end of the antisense strand. In other embodiments, the RNAi agent for inhibiting ACVR2B gene expression comprises nucleotide overhangs at the 3'-end of the sense strand and the 3'-end of the antisense strand. In some embodiments, the RNAi agent for inhibiting ACVR2B gene expression comprises only a nucleotide overhang at the 5'-end of the sense strand. In some embodiments, the RNAi agent for inhibiting ACVR2B gene expression comprises only a nucleotide overhang at the 3'-end of the sense strand. In some embodiments, the RNAi agent for inhibiting ACVR2B gene expression comprises only a nucleotide overhang at the 3'-end of the antisense strand. In some embodiments, the RNAi agent for inhibiting ACVR2B gene expression comprises only a nucleotide overhang at the 5'-end of the antisense strand. In some embodiments, the RNAi agent for inhibiting ACVR2B gene expression comprises only a nucleotide overhang at the 5'-end of the sense strand.

The RNAi agent for inhibiting ACVR2B gene expression may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent for inhibiting ACVR2B gene expression comprises a nucleotide overhang at the 3'-end of the sense strand, and blunt ends at the 5'-end of the sense strand and the 3'-end of the antisense strand. In other embodiments, the RNAi agent for inhibiting ACVR2B gene expression comprises a nucleotide overhang at the 3'-end of the antisense strand, and blunt ends at the 5'-end of the antisense strand and the 3'-end of the sense strand.

Specifically, by way of example, in one embodiment, the RNAi agent for inhibiting ACVR2B gene expression comprises: (i) a sense strand of 19 nucleotides in length, (ii) an antisense strand of 21 nucleotides in length, wherein the strands form a duplex region equal to the length of the sense strand. In another embodiment, the RNAi agent for inhibiting ACVR2B gene expression comprises: (i) a sense strand of 21 nucleotides in length, (ii) an antisense strand of 23 nucleotides in length, wherein the strands form a duplex region equal to the length of the sense strand.

In some embodiments, an RNAi agent for inhibiting ACVR2B gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, and wherein the RNAi agent comprises one overhang and one blunt end, the overhang preferably having 2 unpaired nucleotides.

In some embodiments, an RNAi agent for inhibiting ACVR2B gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting ACVR2B gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting ACVR2B gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, wherein the sense strand is 19 to 21 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting ACVR2B gene expression in cells is provided, comprising a sense strand and an antisense strand that form a duplex region of 19 base pairs in length, wherein the antisense strand is 21 nucleotides in length and consists of at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, the present invention provides an RNAi agent for inhibiting ACVR2B gene expression in cells, comprising a sense strand and an antisense strand that form a duplex region of 19 base pairs in length, wherein the antisense strand is 21 nucleotides in length and consists of any nucleotide sequence selected from SEQ ID NOs: 64 to 94, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In preferred embodiments, the present invention provides an RNAi agent for inhibiting ACVR2B gene expression in cells, comprising a sense strand and an antisense strand that form a duplex region, wherein the antisense strand comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, and wherein the sense strand comprises at least 15 (e.g., 15, 16, 17, 18, or 19) contiguous nucleotides of any nucleotide sequence selected from SEQ ID NOs: 33 to 63 and nucleotide sequences having 1, 2, or 3 nucleotide differences therefrom.

In preferred embodiments, the present invention provides an RNAi agent for inhibiting ACVR2B gene expression in cells, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 64 to 94 and nucleotide sequences having 1, 2, or 3 nucleotide differences therefrom, and wherein the sense strand is no more than 21 nucleotides in length and comprises at least 15 (e.g., 15, 16, 17, 18, or 19) contiguous nucleotides of any nucleotide sequence selected from SEQ ID NOs: 33 to 63 and nucleotide sequences having 1, 2, or 3 nucleotide differences therefrom.

In preferred embodiments, the present invention provides an RNAi agent for inhibiting ACVR2B gene expression in cells, comprising a sense strand and an antisense strand that form a duplex region, wherein the antisense strand is no more than 23 nucleotides in length and comprises any nucleotide sequence selected from SEQ ID NOs: 64 to 94, and wherein the sense strand is no more than 21 nucleotides in length and comprises any nucleotide sequence selected from SEQ ID NOs: 33 to 63.

In preferred embodiments, the present invention provides an RNAi agent for inhibiting ACVR2B gene expression in a cell, comprising a sense strand and an antisense strand that form a duplex region, wherein:
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:79 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:80 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:81 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:82 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:83 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:84 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:85 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:86 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:87 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:88 or a nucleotide sequence having 1, 2, or 3 nucleotide differences therefrom;
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:89 or a nucleotide sequence having 1, 2, or 3 nucleotide differences therefrom;
the sense strand comprises or consists of a sequence set forth in SEQ ID NO:59 or a nucleotide sequence having 1, 2, or 3 nucleotide differences therefrom, and the antisense strand comprises or consists of a sequence set forth in SEQ ID NO:90 or a nucleotide sequence having 1, 2, or 3 nucleotide differences therefrom;
the sense strand comprises or consists of a sequence set forth in SEQ ID NO:60 or a nucleotide sequence having 1, 2, or 3 nucleotide differences therefrom, and the antisense strand comprises or consists of a sequence set forth in SEQ ID NO:91 or a nucleotide sequence having 1, 2, or 3 nucleotide differences therefrom;
the sense strand comprises or consists of a sequence set forth in SEQ ID NO:61 or a nucleotide sequence having 1, 2, or 3 nucleotide differences therefrom, and the antisense strand comprises or consists of a sequence set forth in SEQ ID NO:92 or a nucleotide sequence having 1, 2, or 3 nucleotide differences therefrom;
the sense strand comprises or consists of a sequence set forth in SEQ ID NO:62 or a nucleotide sequence having 1, 2, or 3 nucleotide differences therefrom, and the antisense strand comprises or consists of a sequence set forth in SEQ ID NO:93 or a nucleotide sequence having 1, 2, or 3 nucleotide differences therefrom;
the sense strand comprises or consists of a sequence set forth in SEQ ID NO:63 or a nucleotide sequence having 1, 2, or 3 nucleotide differences therefrom, and the antisense strand comprises or consists of a sequence set forth in SEQ ID NO:94 or a nucleotide sequence having 1, 2, or 3 nucleotide differences therefrom.

In preferred embodiments, the present invention provides an RNAi agent for inhibiting ACVR2B gene expression, comprising a sense strand and an antisense strand that form a complementary duplex region, wherein the sense strand and antisense strand pair to form any one of the duplexes 6102, 6103, 6104, 6105, 6106, 6107, 6108, 6109, 6112, 6113, 6115, 6116, 6117, 6119, 6120, 6121, 6122, 6123, 6124, 6125, 6127, 6128, 6129, 6130, 6131, 6132, 6134, 6135, 6137, 6138, and 6140 as described herein.

### Nucleotide-Modified RNAi Agents

For any of the embodiments of the RNAi agents of the present invention described in the section "RNAi Agents For Inhibiting ACVR2B Gene Expression", the sense strand and/or antisense strand of the RNAi agent may comprise at least one modified nucleotide.

In some embodiments, the sense strand and the antisense strand of the RNAi agent for inhibiting ACVR2B gene expression provided by the present invention each comprise at least one modified nucleotide.

In some embodiments, each of the nucleotides in the sense strand and the antisense strand of the RNAi agent for inhibiting ACVR2B gene expression provided by the present invention is modified.

In any of the above embodiments, the modified nucleotide is independently selected from the group consisting of: 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof.

In preferred embodiments, the modified nucleotides are independently selected from the group consisting of 2'-O-methyl-modified nucleotides, 2'-fluoro-modified nucleotides, nucleotides comprising phosphorothioate internucleoside linkages, and combinations thereof. In preferred embodiments, every nucleotide of the sense strand and/or the antisense strand of the RNAi agent is modified. In preferred embodiments, every nucleotide of the sense strand and/or the antisense strand of the RNAi agent is modified, and the modified nucleotides are independently selected from the group consisting of 2'-O-methyl-modified nucleotides, 2'-fluoro-modified nucleotides, nucleotides comprising phosphorothioate internucleoside linkages, and combinations thereof.

In preferred embodiments, each of the nucleotides in the sense strand and the antisense strand of the RNAi agent for inhibiting ACVR2B gene expression provided by the present invention is modified, and the modification is selected from the group consisting of STC(Alnylam), ESC(Alnylam), Advanced ESC(Alnylam), ESC+(Alnylam), AD1-3(Arrowhead), AD5(Arrowhead) and GalXC(Dicerna) (see, e.g., Hu B, Zhong L, Weng Y, et al. Therapeutic siRNA: state of the art. Signal Transduct Target Ther. 2020; 5(1):101. Published 2020 Jun 19. doi:10.1038/s41392-020-0207-x).

In preferred embodiments, the 5'-terminal nucleotide of the antisense strand of the RNAi agent for inhibiting ACVR2B gene expression provided by the present invention contains a phosphate or phosphate analog modification, preferably 5'-VP (see Parmar R, Willoughby JL, Liu J, et al. 5'-(E)-Vinylphosphonate: A Stable Phosphate Mimic Can Improve the RNAi Activity of siRNA-GalNAc Conjugates. Chembiochem. 2016;17(11):985-989).

In some embodiments, in the RNAi agent for inhibiting ACVR2B gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent for inhibiting ACVR2B gene expression provided by the present invention, each of the nucleotides of the antisense strand is modified, and in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, in the RNAi agent for inhibiting ACVR2B gene expression provided by the present invention, each of the nucleotides of the antisense strand is modified, and in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, in the RNAi agent for inhibiting ACVR2B gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage. In preferable embodiments, the phosphorothioate internucleotide linkage is present at one or more of the following locations: (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand. In preferable embodiments, the phosphorothioate internucleotide linkage is present (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand.

In some embodiments, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides.

For example, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides.

For example, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In preferred embodiments, the sense strand has at least one phosphorothioate internucleoside linkage. In preferred embodiments, the phosphorothioate internucleoside linkage is present in one or more of the following positions: (i) between nucleotide position 1 and nucleotide position 2 at the 5' end of the sense strand; (ii) between nucleotide position 2 and nucleotide position 3 at the 5' end of the sense strand; (iii) between nucleotide position 1 and nucleotide position 2 at the 3' end of the sense strand; and (iv) between nucleotide position 2 and nucleotide position 3 at the 3' end of the sense strand. In more preferred embodiments, the phosphorothioate internucleoside linkages are present between nucleotide position 1 and nucleotide position 2 at the 5' end of the sense strand; and between nucleotide position 2 and nucleotide position 3 at the 5' end of the sense strand. In more preferred embodiments, the phosphorothioate internucleoside linkages are present between nucleotide position 1 and nucleotide position 2 at the 5' end of the sense strand; between nucleotide position 2 and nucleotide position 3 at the 5' end of the sense strand; between nucleotide position 1 and nucleotide position 2 at the 3' end of the sense strand; and between nucleotide position 2 and nucleotide position 3 at the 3' end of the sense strand.

In preferred embodiments, the antisense strand has at least one phosphorothioate internucleoside linkage. Preferably, the phosphorothioate internucleoside linkage is present in one or more of the following: between nucleotide position 1 and nucleotide position 2 at the 5' end of the antisense strand; between nucleotide position 2 and nucleotide position 3 at the 5' end of the antisense strand; between nucleotide position 1 and nucleotide position 2 at the 3' end of the antisense strand; and between nucleotide position 2 and nucleotide position 3 at the 3' end of the antisense strand.

Accordingly, in some embodiments, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and/or between nucleotide positions 2 and 3 at the 5'-end of the sense strand.

In some embodiments, in the RNAi agent for inhibiting ACVR2B gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and between nucleotide positions 2 and 3 at the 5'-end of the sense strand.

### Ligand-Conjugated RNAi Agents

For any of the embodiments of the RNAi agent of the present invention described in the preceding sections entitled "RNAi Agents For Inhibiting ACVR2B Gene Expression" and "Nucleotide-modified RNAi agents", the RNAi agent may comprise a ligand. As used herein, a "ligand" refers to any compound or molecule capable of interacting, directly or indirectly, with another compound or molecule. The interaction of a ligand with another compound or molecule may elicit a biological response (e.g., initiation of a signal transduction cascade, induction of receptor-mediated endocytosis) or may simply be a physical attachment. A ligand may alter one or more properties of the attached double-stranded RNA molecule, such as the pharmacodynamics, pharmacokinetics, binding, absorption, cellular distribution, cellular uptake, charge, and/or clearance of the RNA molecule.

The ACVR2B gene is expressed in the various cells and tissues. Therefore, in certain embodiments, it is desirable to specifically deliver the ASO or RNAi agents of the present invention to hepatocytes. Accordingly, in certain embodiments, the ligand targets the specific delivery of the ASO or RNAi agent to hepatocytes using various methods described in more detail below. In certain embodiments, the ASO or RNAi agent is targeted to hepatocytes with a ligand that binds the surface-expressed asialoglycoprotein receptor (ASGR) or a component thereof (e.g., ASGR1, ASGR2).

In some embodiments, the ASO or RNAi agent can be specifically targeted to the liver by using a ligand that binds or interacts with a protein expressed on the surface of hepatocytes. For example, in certain embodiments, the ligand can comprise an antigen-binding protein (e.g., an antibody or binding fragment thereof such as Fab, scFv) that specifically binds a receptor expressed on hepatocytes, such as the asialoglycoprotein receptor and the LDL receptor. In one particular embodiment, the ligand comprises an antibody or binding fragment thereof that specifically binds ASGR1 and/or ASGR2. In another embodiment, the ligand comprises a Fab fragment of an antibody that specifically binds ASGR1 and/or ASGR2. In another embodiment, the ligand comprises a single-chain variable antibody fragment (scFv fragment) of an antibody that specifically binds ASGR1 and/or ASGR2. Exemplary antibodies that specifically bind ASGR1 and binding fragments thereof, which can serve as ligands for targeting the RNAi agents of the present invention to the liver, are described in WO 2017/058944, which is incorporated herein by reference in its entirety. Other antibodies or binding fragments thereof that specifically bind ASGR1, the LDL receptor, or other liver surface-expressed proteins suitable for use as ligands in the RNAi agents of the present invention are available from commercial sources.

In some embodiments, when used for extrahepatic delivery, including but not limited to, the central nervous system (CNS) (e.g., brain, spinal cord, or eye), muscle, lung, or adipose tissue, the RNAi agent may comprise one or more lipophilic groups conjugated to one or more positions of the antisense strand and/or sense strand. For example, the lipophilic group may be linked to the antisense strand and/or sense strand via a nucleobase, sugar moiety, or internucleoside linkage. In some embodiments, a lipophilic group having a phosphoramidite group is coupled to the 3' end or the 5' end of the sense strand or antisense strand in the final synthesis cycle. In some embodiments, the lipophilic group has an octanol-water partition coefficient exceeding 0, 1, 1.5, 2, 3, 4, 5, or 10. In some embodiments, the ligand is a lipophilic group, which is preferably selected from: lipids, vitamins, steroids, C₅-C₃₀ saturated or unsaturated fatty acids, C₅-C₃₀ alkyl groups, and polypeptides comprising at least one positively charged amino acid residue; said lipophilic group is more preferably selected from cholesterol, C₁₆ saturated or unsaturated fatty acids, C₁₆ alkyl groups, C₂₂ saturated or unsaturated fatty acids, or C₂₂ alkyl groups. Suitable lipophilic groups may be aliphatic, alicyclic, polyalicyclic compounds, steroids, linear or branched aliphatic hydrocarbon groups.

Exemplary lipophilic groups include, for example, lipophilic groups Y132 to Y135, Y158, Y165 to Y168, L10, L57, L321, L322, Q361 to Q367, Q361s to Q367s, Q370, Q377 to Q379, Q383, etc., as described in WO 2021/092371. Other ligands that may be conjugated to the antisense oligonucleotide or RNAi agent of the present invention can be found in WO 2017/053995, WO 2019/217459, WO 2021/092371, WO 2017/053995, WO 2010/039548, WO 2023/064530, WO 2022/213118, and WO 2019/079386, each of which is incorporated herein by reference in its entirety.

In certain embodiments, the ligand comprises a carbohydrate. A "carbohydrate" refers to a compound composed of one or more monosaccharide units having at least 6 carbon atoms (which can be linear, branched, or cyclic) with oxygen, nitrogen, or sulfur atoms attached to each carbon atom. Carbohydrates include, but are not limited to, sugars (e.g., monosaccharides, disaccharides, trisaccharides, tetrasaccharides, and oligosaccharides containing about 4, 5, 6, 7, 8, or 9 monosaccharide units) and polysaccharides (such as starch, glycogen, cellulose, and polysaccharide gums). In some embodiments, the carbohydrate incorporated into the ligand is selected from pentoses, hexoses, or heptoses, and disaccharides and trisaccharides including such monosaccharide units. In other embodiments, the carbohydrate incorporated into the ligand is an amino sugar, such as galactosamine, glucosamine, N-acetylgalactosamine, and N-acetylglucosamine.

In some embodiments, the ligand comprises a hexose or a hexosamine. The hexose can be selected from glucose, galactose, mannose, fucose, or fructose. The hexosamine can be selected from fructosamine, galactosamine, glucosamine, or mannosamine. In certain embodiments, the ligand comprises glucose, galactose, galactosamine, or glucosamine. In one embodiment, the ligand comprises glucose, glucosamine, or N-acetylglucosamine. In another embodiment, the ligand comprises galactose, galactosamine, or N-acetylgalactosamine. In particular embodiments, ligands comprising glucose, galactose, and N-acetylgalactosamine (GalNAc) are particularly effective in targeting RNA to hepatocytes, as these ligands bind the ASGR expressed on the surface of hepatocytes. Examples of GalNAc- or galactose-containing ligands that can be incorporated into the RNAi agents of the present invention are described in U.S. Pat. Nos. 7,491,805, 8,106,022, and 8,877,917; U.S. Patent Application Publication No. US 20030130186; and WIPO Publication No. WO 2013/166155, all of which are incorporated herein by reference in their entirety.

In certain embodiments, the ligand comprises a multivalent carbohydrate moiety. As used herein, a "multivalent carbohydrate moiety" refers to a moiety comprising two or more carbohydrate units capable of independently binding or interacting with other molecules. For example, a multivalent carbohydrate moiety includes two or more binding domains composed of carbohydrates that can bind to two or more different molecules or two or more different sites on the same molecule. The "valency" of a carbohydrate moiety indicates the number of individual binding domains within the carbohydrate moiety. For example, the terms "monovalent," "bivalent," "trivalent," and "tetravalent" relative to a carbohydrate moiety refer to carbohydrate moieties having one, two, three, and four binding domains, respectively. A multivalent carbohydrate moiety can comprise a multivalent lactose moiety, a multivalent galactose moiety, a multivalent glucose moiety, a multivalent N-acetylgalactosamine moiety, a multivalent N-acetylglucosamine moiety, a multivalent mannose moiety, or a multivalent fucose moiety. In some embodiments, the ligand comprises a multivalent galactose moiety. In other embodiments, the ligand comprises a multivalent N-acetylgalactosamine moiety. In these and other embodiments, the multivalent carbohydrate moiety can be bivalent, trivalent, or tetravalent. In such embodiments, the multivalent carbohydrate moiety can be bi-branched or tri-branched. In one particular embodiment, the multivalent N-acetylgalactosamine moiety is trivalent or tetravalent. In another particular embodiment, the multivalent galactose moiety is trivalent or tetravalent. Exemplary trivalent and tetravalent GalNAc-containing ligands for incorporation into the ASO or RNAi agents of the present invention are described in detail below.

The ligand may also comprise an integrin ligand, for example, a ligand that specifically binds to an integrin, including but not limited to αVβ6. Suitable integrin ligands include, for example, those integrin ligands mentioned in WO2019089765A1 or WO2022056286A1, which are incorporated herein by reference in their entirety.

The ligand may also comprise a transferrin receptor 1 (TfR1) ligand, such as an anti-TfR1 antibody or polypeptide. Suitable TfR1 ligands include, for example, TfR1 antibodies described in WO2022147209A1 or WO2021154476A1, or bicyclic peptide ligands described in WO2022101633A1, and these patent documents are incorporated herein by reference in their entirety.

The ligand can be attached or conjugated to the RNA molecule of the ASO or RNAi agent directly or indirectly. For example, in some embodiments, the ligand is directly covalently attached to the ASO ro the sense strand or the antisense strand of the RNAi agent. In other embodiments, the ligand is covalently attached to the ASO or the sense strand or the antisense strand of the RNAi agent via a linker. The ligand can be attached to a nucleobase, sugar moiety, or internucleotide linkage of ASO or the sense strand or antisense strand of the RNAi agent of the present invention.

In some embodiments, the ligand can be attached to 3' or 5'-end of the sense strand or antisense strand. In certain embodiments, the ligand is covalently attached to 5'-end of the sense strand. In such embodiments, the ligand is attached to 5'-terminal nucleotide of the sense strand. In these and other embodiments, the ligand is attached at 5'-position of 5'-terminal nucleotide of the sense strand. In other embodiments, the ligand is covalently attached to 3'-end of the sense strand. For example, in some embodiments, the ligand is attached to 3'-terminal nucleotide of the sense strand. In certain such embodiments, the ligand is attached at 3'-position of 3'-terminal nucleotide of the sense strand. In alternative embodiments, the ligand is attached near 3'-end but prior to one or more terminal nucleotides (i.e., one, two, three, or four nucleotides from the terminus). In some embodiments, the ligand is attached at the 2'-position of the sugar of 3'-terminal nucleotide of the sense strand. In other embodiments, the ligand is attached at the 2'-position of the sugar of 5'-terminal nucleotide of the sense strand.

In certain embodiments, the ligand is attached to the sense strand or antisense strand via a linker. A "linker" refers to an atom or group of atoms that covalently attaches the ligand to the polynucleotide component of the ASO or RNAi agent. The linker can have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker can comprise a bifunctional linking moiety, typically comprising an alkyl group with two functional groups. One functional group is chosen to bind to the compound of interest (e.g., the sense strand or antisense strand of the RNAi agent), while the other functional group is chosen to bind to essentially any selected group, such as the ligands described herein. In certain embodiments, the linker comprises an oligomer of a chain structure or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes an amino group, a hydroxyl group, a carboxylic acid, a thiol, an unsaturated bond (e.g., a double or triple bond), and the like.

Linkers suitable for attaching a ligand to the ASO or sense strand or antisense strand of the RNAi agent of the present invention include, but are not limited to, pyrrolidine, 8-amino-3,6-dioxaoctanoic acid, succinimidyl-4-(N-maleimidomethyl)cyclohexane-1-carboxylate, 6-aminohexanoic acid, substituted C₁-C₂₀ alkyl, substituted or unsubstituted C₂-C₂₀ alkenyl, or substituted or unsubstituted C₂-C₂₀ alkynyl. Preferred substituents for such linkers include, but are not limited to, hydroxyl, amino, alkoxy, carboxyl, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved to release the two parts linked together by the linker upon entry into a target cell. In some embodiments, the cleavable linker is cleaved at least 10, 20, 30, 40, 50, 60, 70, 80, 90 times or more, or at least 100 times faster in the target cell or under a first reference condition (which can, for example, be selected to mimic or represent intracellular conditions) than in the blood of a subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted. For example, a liver-targeting ligand can be linked to the RNA molecule via a linker comprising of an ester group. Hepatocytes are rich in esterases, so the linker is cleaved more efficiently in hepatocytes than in cell types not rich in esterases. Other cell types rich in esterases include cells of the lung, renal cortex, and testis. When targeting cells rich in peptidases, such as hepatocytes and synoviocytes, linkers containing peptide bonds can be used.

Other types of linkers suitable for attaching ligands to the sense strand or antisense strand in the RNAi agents of the present invention are known in the art, such as those described in U.S. Pat. Nos. 7,723,509, 8,017,762, 8,828,956, 8,877,917, and 9,181,551, all of which are incorporated herein by reference in their entirety.

In certain embodiments, the ligand covalently attached to the ASO or the sense strand or antisense strand of the RNAi agent of the present invention comprises a GalNAc moiety, such as a multivalent GalNAc. In some embodiments, the multivalent GalNAc moiety is a trivalent GalNAc and is attached to 3'-end of the sense strand. In other embodiments, the multivalent GalNAc moiety is a trivalent GalNAc and is attached to 5'-end of the sense strand. In other embodiments, the multivalent GalNAc moiety is a tetravalent GalNAc moiety and is attached to 3'-end of the sense strand. In other embodiments, the multivalent GalNAc moiety is a tetravalent GalNAc moiety and is attached to 5'-end of the sense strand.

In some embodiments, the ligand is L96, NAG25, and NAG37, having the formulas shown below, respectively, wherein the wave line represents a site to which the ASO or the sense strand or antisense strand of the RNAi agent is attached.

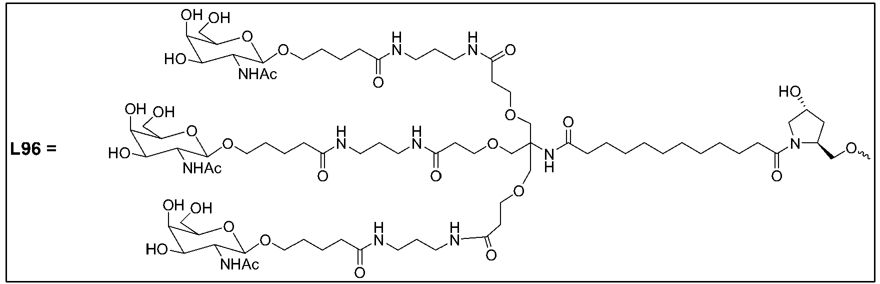

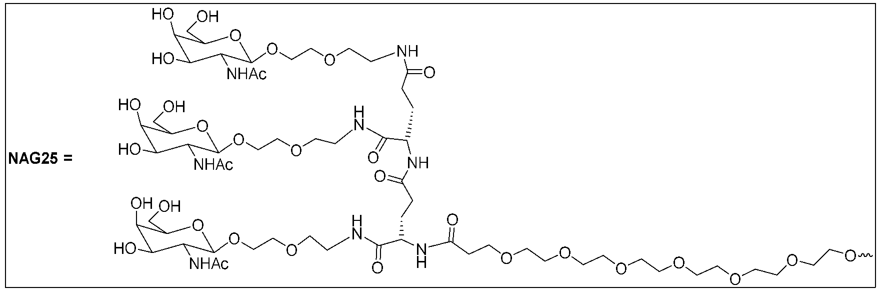

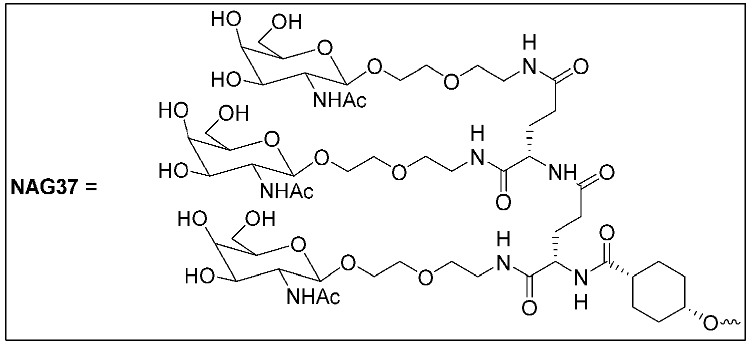

### Pharmaceutical Compositions

The present invention also encompasses pharmaceutical compositions and formulations comprising the antisense oligonucleotides or RNAi agents described herein and a pharmaceutically acceptable carrier, excipient, or diluent. Such compositions and formulations can be used to reduce the expression of the ACVR2B gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery. In such embodiments, the pharmaceutical composition may comprise a targeting ligand (e.g., a GalNAc-containing or antibody-containing ligand as described herein).

In some embodiments, the pharmaceutical composition comprises an effective amount of the antisense oligonucleotides or RNAi agent described herein. An "effective amount" refers to an amount sufficient to produce a beneficial or desired clinical outcome. In some embodiments, the effective amount is an amount sufficient to reduce the expression of the ACVR2B gene in a specific tissue or cell type of the patient (e.g., the liver or hepatocytes). The effective amount of the antisense oligonucleotides or RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient's body size, age, and general condition, the type of disease to be treated (e.g., myocardial infarction, coronary artery disease, peripheral artery disease, stroke), the specific antisense oligonucleotides or RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the antisense oligonucleotides or RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid (Baxter International Inc.), Liposyn (Abbott Pharmaceuticals), Lipsyn II (Hospira), Liposyn III (Hospira), Nutrilipid (B. Braun Medical Inc.), and other similar fat emulsions. A preferred colloidal system for use as an *in vivo* delivery vehicle is the liposome (i.e., an artificial membrane vesicle). The RNAi agents of the present invention can be encapsulated within liposomes or can form complexes therewith, particularly with cationic liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Liposomal formulations are particularly suitable for topical administration, with liposomes conferring several advantages over alternative formulations. Such advantages include reduced incidence of adverse effects associated with high systemic absorption of administered therapeutics, enhanced accumulation of administered therapeutics at desired target sites, and capability for dermal delivery of antisense oligonucleotides or RNAi agents. In some embodiments, liposomes are employed to deliver RNAi agents to epidermal cells while concurrently enhancing permeation of said antisense oligonucleotides or RNAi agents into dermal tissues, e.g., within the skin.

The antisense oligonucleotides or RNAi agents of the present invention may be fully encapsulated within lipid-based formulations, such as LNPs or other nucleic acid-lipid particles. As used herein, the term "LNP" refers to stable nucleic acid-lipid particles. LNPs typically comprise a cationic lipid, a non-cationic lipid, and a lipid that prevents particle aggregation (e.g., a PEG-lipid conjugate). LNPs are particularly useful for systemic applications as they exhibit prolonged circulation times following intravenous (i.v.) injection and accumulate at distal sites (e.g., sites physically separated from the administration site). LNPs include "pSPLP," which comprise encapsulated condensing agent-nucleic acid complexes as described in WO00/03683. The LNP particles of the present invention generally possess a mean diameter ranging from about 50 nm to about 150 nm, more typically from about 60 nm to about 130 nm, more typically from about 70 nm to about 110 nm, and most typically from about 70 nm to about 90 nm, and are substantially non-toxic. Additionally, when nucleic acids are present within the nucleic acid-lipid particles of the invention, they demonstrate resistance to nuclease degradation in aqueous solutions. Nucleic acid-lipid particles and methods for their preparation are disclosed in, for example, U.S. Pat. Nos. 5,976,567; 5,981,501; 6,534,484; 6,586,410; 6,815,432; U.S. Publication No. 2010/0324120; and WO 96/40964. In one embodiment, the lipid-to-drug ratio (mass/mass basis) (e.g., lipid-to-RNAi agent ratio) will range from about 1:1 to about 50:1, about 1:1 to about 25:1, about 3:1 to about 15:1, about 4:1 to about 10:1, about 5:1 to about 9:1, or about 6:1 to about 9:1.

Cationic lipids may include, for example, N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dioleyloxy-N,N-dimethylaminopropane (DLinDMA), 1,2-dilinoleyloxy-N,N-dimethylaminopropane (DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinolenyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleoyloxy-3-(N-methylpiperazinyl)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-(bis(2-hydroxydodecyl)amino)ethyl)(2-hydroxydodecyl)amino)ethyl)piperazin-1-yl)ethylazanediyl)didodecan-2-ol (Tech G1), or mixtures thereof. The cationic lipid may constitute about 20 mol% to about 50 mol%, or about 40 mol% of the total lipid present in the particle.

In some embodiments, the compound 2,2-dilinoleyl-4-dimethylaminomethyl-[1,3]-dioxolane may be utilized in the preparation of lipid-siRNA nanoparticles. In certain embodiments, the lipid-siRNA particles comprise 40% 2,2-dilinoleyl-4-dimethylaminomethyl-[1,3]-dioxolane : 10% DSPC : 40% cholesterol : 10% PEG-C-DOMG (mol%), with a particle size of 63.0 ± 20 nm and an siRNA-to-lipid ratio of 0.027.

Ionizable/non-cationic lipids may be anionic lipids or neutral lipids, including but not limited to distearoylphosphatidylcholine (DSPC), dioleoylphosphatidylcholine (DOPC), dipalmitoylphosphatidylcholine (DPPC), dioleoylphosphatidylglycerol (DOPG), dipalmitoyl (DP), dioleoylphosphatidylethanolamine (DOPE), palmitoyloleoylphosphatidylcholine (POPC), palmitoyloleoylphosphatidylethanolamine (POPE), N-(4-(p-maleimidophenyl)butyryl)-dioleoylphosphatidylethanolamine (DOPE-mal), dipalmitoylphosphatidylethanolamine (DPPE), dimyristoylphosphatidylethanolamine (DMPE), distearoylphosphatidylethanolamine (DSPE), 16-O-monomethyl PE, 16-O-dimethyl PE, 18-1-trans PE, 1-stearoyl-2-oleoyl-phosphatidylethanolamine (SOPE), cholesterol, or mixtures thereof. The non-cationic lipid may constitute about 5 mol% to about 90 mol%, about 10 mol%, or about 58 mol% (when cholesterol is included) of the total lipid present in the particle.

Lipids conjugated to prevent particle aggregation may include, for example, polyethylene glycol (PEG)-lipids, comprising but not limited to PEG-diacylglycerol (PEG-DAG), PEG-dialkoxypropyl (PEG-DAA), PEG-phospholipid, PEG-ceramide (PEG-Cer), or mixtures thereof. PEG-DAA conjugates may include, for instance, PEG-didodecyloxypropyl (C12), PEG-dimyristyloxypropyl (C14), PEG-dipalmityloxypropyl (C14), or PEG-distearoyloxypropyl (C18). The lipid conjugated to prevent particle aggregation may constitute 0 mol% to about 20 mol%, or about 2 mol% of the total lipid present in the particle. In some embodiments, the nucleic acid-lipid particles further comprise cholesterol, for example constituting about 10 mol% to about 60 mol%, or about 48 mol% of the total lipid present in said particle.

In one embodiment, the lipidoid ND98·4HCl (molecular weight 1487) (see U.S. Patent Application No. 12/056,230, incorporated herein by reference in its entirety), cholesterol (Sigma-Aldrich), and PEG-ceramide C16 (Avanti Polar Lipids) may be utilized to prepare lipid-dsRNA nanoparticles (i.e., LNP01 particles). Each stock solution in ethanol may be prepared as follows: ND98 at 133 mg/mL; cholesterol at 25 mg/mL; PEG-ceramide C16 at 100 mg/mL. The stock solutions of ND98, cholesterol, and PEG-ceramide C16 may then be mixed at a mol ratio of, for example, 42:48:10. The combined lipid solution may be mixed with aqueous siRNA (e.g., in sodium acetate buffer at pH 5) such that the final ethanol concentration is about 35-45% and the final sodium acetate concentration is about 100-300 mM. Lipid-siRNA nanoparticles typically form spontaneously upon mixing.

Other exemplary lipid-siRNA formulations are available from *e.g.* WO2009/127060(SNALP), PCT/US2010/022614(XTC), US 2010/0324120(MC3), PCT/US09/63933(ALNY-100) and WO2010/129709(C12-200).

In some embodiments, the antisense oligonucleotide or RNAi agent of the present invention may be conjugated to a cell-penetrating peptide (CPP), wherein said CPP may comprise at least one positively charged amino acid or ionizable amino acid, such as arginine, to form a peptide-oligonucleotide conjugate. Suitable CPPs for forming peptide-oligonucleotide conjugates may be found, for example, in those described in WO2022213118A1 or WO2019079386A1, which are incorporated herein by reference in their entirety.

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water, ethanol, polyhydric alcohols (e.g., glycerol, propylene glycol, liquid polyethylene glycols, and the like), suitable mixtures thereof, and vegetable oils. Appropriate fluidity may be maintained, for example, using a coating material such as lecithin, by maintaining required particle size in dispersions, or by employing surfactants. Prevention of microbial action may be achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the antisense oligonucleotide or RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the antisense oligonucleotide or RNAi agent described herein and sterile water (e.g., Water for Injection, WFI), or consists essentially thereof. In yet other embodiments, the pharmaceutical composition of the invention comprises the antisense oligonucleotide or RNAi agent described herein and Phosphate-Buffered Saline (PBS), or consists essentially thereof.

In certain embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

### Therapeutic Methods and Uses

The present invention provides a method for reducing or inhibiting the expression of ACVR2B gene in a cell (e.g., a hepatocyte) by contacting the cell with any of the antisense oligonucleotides or RNAi agents described herein. The cell can be *in vitro* or *in vivo*. ACVR2B gene expression can be assessed by measuring the amount or level of ACVR2B mRNA or ACVR2B protein. The reduction in ACVR2B expression in cells or animals treated with the antisense oligonucleotides or RNAi agents of the invention can be determined relative to ACVR2B expression in cells and animals not treated with the antisense oligonucleotide or RNAi agent or treated with a control antisense oligonucleotide or RNAi agent. For example, in some embodiments, the reduction in ACVR2B expression is assessed by (a) measuring the amount or level of ACVR2B mRNA in hepatocytes treated with an antisense oligonucleotide or RNAi agent of the invention, (b) measuring the amount or level of ACVR2B mRNA in hepatocytes treated with a control RNAi agent (e.g., an RNAi agent targeting an RNA molecule not expressed in hepatocytes or an antisense oligonucleotide or RNAi agent having a nonsense or scrambled sequence) or no antisense oligonucleotide or RNAi agent, and (c) comparing the ACVR2B mRNA level measured in the treated cells of (a) with the ACVR2B mRNA level in the control cells of (b). The ACVR2B mRNA levels in the treated and control cells may be normalized to the RNA level of a control gene (e.g., 18S ribosomal RNA or a housekeeping gene) prior to comparison. ACVR2B mRNA levels can be measured by various methods, including Northern blot analysis, nuclease protection assay, fluorescent in situ hybridization (FISH), reverse transcriptase (RT)-PCR, real-time RT-PCR, quantitative PCR, droplet digital PCR, and the like.

In some embodiments, the method for assessing ACVR2B expression levels is performed *in vitro* in cells that naturally express the ACVR2B gene (e.g., hepatocytes) or cells that have been engineered to express ACVR2B.

In other embodiments, the method for assessing ACVR2B expression levels is performed *in vivo*. The antisense oligonucleotide or RNAi agent and any control antisense oligonucleotide or RNAi agent can be administered to an animal (e.g., a transgenic animal expressing the ACVR2B gene or a non-human primate), and ACVR2B mRNA or serum ACVR2B protein levels are assessed in liver tissue harvested from the animal post-treatment. Alternatively, or additionally, biomarkers or functional phenotypes associated with ACVR2B expression can be assessed in the treated animal. For example, ACVR2B protein is the expression product of ACVR2B mRNA in serum or plasma. Therefore, the serum or plasma levels of ACVR2B protein can be measured in an animal treated with an antisense oligonucleotide or RNAi agent of the invention to assess the functional efficacy of reducing ACVR2B expression.

In certain embodiments, the expression of ACVR2B in hepatocytes is reduced by the antisense oligonucleotide or RNAi agent of the invention by at least 40%, at least 45%, or at least 50%. In some embodiments, the expression of ACVR2B in hepatocytes is reduced by the RNAi agent of the invention by at least 60%, at least 65%, at least 70%, at least 75%, at least 80%, or at least 85%. In other embodiments, the expression of ACVR2B in hepatocytes is reduced by the antisense oligonucleotide or RNAi agent of the invention by about 90% or more, e.g., 91%, 92%, 93%, 94%, 95%, 96%, 97%, 98%, 99%, or more. The percentage reduction in ACVR2B expression can be measured by any of the methods described herein, as well as other methods known in the art.

The present invention provides methods for reducing or inhibiting the expression of the ACVR2B gene, thereby reducing or inhibiting the production of ACVR2B protein, in a patient in need thereof, as well as methods for treating or preventing a disease or disorder associated with ACVR2B expression or activity. A "disease or disorder associated with ACVR2B expression" refers to a disease or disorder characterized by altered levels of ACVR2B expression, or a condition where elevated levels of ACVR2B expression are associated with an increased risk of developing the disease or disorder. In certain embodiments, the RNAi agents of the invention are particularly useful for treating or preventing metabolic diseases and reducing circulating levels of ACVR2B.

Diseases and disorders associated with ACVR2B expression that can be treated or prevented according to the methods of the invention include, but are not limited to, muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.

In certain embodiments, the present invention provides a method for reducing ACVR2B expression in a patient in need thereof, comprising administering to the patient any antisense oligonucleotide or RNAi agent described herein. Preferably, the level of ACVR2B expression in the patient's hepatocytes is reduced following administration of the antisense oligonucleotide or RNAi agent, compared to the level of ACVR2B expression in a patient not receiving the antisense oligonucleotide or RNAi agent, or compared to the level of ACVR2B expression in the patient prior to administration of the antisense oligonucleotide or RNAi agent. In some embodiments, after administration of the antisense oligonucleotide or RNAi agent of the invention, ACVR2B expression in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in ACVR2B expression can be measured by any of the methods described herein, as well as other methods known in the art. In certain embodiments, the percentage reduction in ACVR2B expression is determined by assessing the level of ACVR2B protein in the patient's serum or plasma according to the methods described herein.

In certain embodiments, a patient in need of reducing ACVR2B expression is a patient diagnosed with or at risk of developing a disease mediated by aberrant expression levels of ACVR2B. Accordingly, the present invention includes a method of treating or preventing a disease mediated by aberrant expression levels of ACVR2B in a patient in need thereof by administering any antisense oligonucleotide or RNAi agent of the present invention. In some embodiments, the present invention includes use of any antisense oligonucleotide or RNAi agent described herein in the manufacture of a medicament for treating or preventing a disease mediated by aberrant expression levels of ACVR2B in a patient in need thereof. In other embodiments, the present invention provides an ACVR2B-targeting antisense oligonucleotide or RNAi agent for use in a method of treating or preventing a disease mediated by aberrant expression levels of ACVR2B in a patient in need thereof. As described above, the disease mediated by aberrant expression levels of ACVR2B is, for example, muscle atrophy, muscular dystrophy, obesity, diabetes mellitus, chronic kidney disease, acute kidney injury, post-traumatic and postoperative wound healing, maxillofacial tumors, preeclampsia, liver fibrosis, atherosclerosis, ischemic stroke, and Alzheimer's disease. In one embodiment, the disease is muscle atrophy. In one embodiment, the disease is muscular dystrophy. In one embodiment, the disease is obesity. In one embodiment, the disease is diabetes mellitus. In one embodiment, the disease is chronic kidney disease. In one embodiment, the disease is acute kidney injury. In one embodiment, the disease is post-traumatic and postoperative wound healing. In one embodiment, the disease is maxillofacial tumors. In one embodiment, the disease is preeclampsia. In one embodiment, the disease is liver fibrosis. In one embodiment, the disease is atherosclerosis. In one embodiment, the disease is ischemic stroke. In one embodiment, the disease is Alzheimer's disease.

In certain other embodiments, a patient in need of reducing ACVR2B expression is a patient with elevated levels of ACVR2B. Accordingly, in some embodiments, the present invention provides a method of reducing ACVR2B protein levels in a patient in need thereof by administering to the patient any antisense oligonucleotide or RNAi agent described herein. In some embodiments, the present invention includes use of any antisense oligonucleotide or RNAi agent described herein in the manufacture of a medicament for reducing ACVR2B protein levels in a patient in need thereof. In other embodiments, the present invention provides an ACVR2B-targeting antisense oligonucleotide or RNAi agent for use in a method of reducing ACVR2B protein levels in a patient in need thereof.

In some embodiments of the present invention, the present invention provides use of an agent that reduces the expression of the gene encoding activin type IIB receptor (ACVR2B) in the manufacture of a medicament for treating a disease or disorder treatable by ACVR2B knockdown. In preferred embodiments, said agent is an antisense oligonucleotide and/or an RNAi agent. In preferred embodiments, said disease or disorder is selected from the group consisting of muscle atrophy, muscular dystrophy, obesity, diabetes mellitus, chronic kidney disease, acute kidney injury, post-traumatic and postoperative wound healing, maxillofacial tumors, preeclampsia, liver fibrosis, atherosclerosis, ischemic stroke, and Alzheimer's disease.

Therefore, in some embodiments, the present invention provides use of an antisense oligonucleotide and/or RNAi agent that reduces the expression of the gene encoding activin type IIB receptor (ACVR2B) in the manufacture of a medicament for treating a disease or disorder selected from the group consisting of muscle atrophy, muscular dystrophy, obesity, diabetes mellitus, chronic kidney disease, acute kidney injury, post-traumatic and postoperative wound healing, maxillofacial tumors, preeclampsia, liver fibrosis, atherosclerosis, ischemic stroke, and Alzheimer's disease.

### Sequence Listings

**Table 1. Oligonucleotides targeting ACVR2B (naked sequences and exemplary modified sequences)**

| **Name** | **Position** | **ASO (5'-3')** | **SED ID NO** | **Naked Sequences(5'-3')** | **SED ID NO** |
|---|---|---|---|---|---|
| 6001 -2 | 509-528 | eT*eA*eG*eT*eA*dG*dA*dT*dG*m5dC* dA*m5dC*dT*m5dC*m5dC*m5eC*eG*eT *eG*eT | 18 | TAGTAGAT GCACTCCC GTGT | 3 |
| 6003 -2 | 658-677 | eA*eG*eT*m5eC*eA*dT*m5dC*dT*dA*d G*m5dC*m5dC*dA*dG*m5dC*eA*eG*m 5eC*m5eC*m5eC | 19 | AGTCATCT AGCCAGCA GCCC | 4 |
| 6005 -2 | 842-861 | eA*eG*eT*eG*eA*dG*dT*dA*dG*dG*m5 dC*m5dC*dA*dG*m5dC*eA*m5eC*m5eC *eG*eT | 20 | AGTGAGTA GGCCAGCA CCGT | 5 |
| 6009 -2 | 1244-1263 | eA*eG*eG*eT*eA*dA*dT*m5dC*m5dC*d G*dT*dG*dA*dG*dG*eG*eA*eG*m5eC* m5eC | 21 | AGGTAATC CGTGAGGG AGCC | 6 |
| 6011 -2 | 1365-1384 | eA*eA*eT*eA*eG*dA*m5dC*dG*dG*m5 dC*dT*dT*dG*dT*dG*eG*m5eC*m5eC* m5eC*eT | 22 | AATAGACG GCTTGTGG CCCT | 7 |
| 6012 -2 | 1508-1527 | eA*eT*eG*eT*eA*m5dC*m5dC*dG*dT* m5dC*dT*m5dC*dG*dT*dG*m5eC*m5eC *eT*eA*m5eC | 23 | ATGTACCG TCTCGTGCC TAC | 8 |
| 6017 -2 | 515-534 | eG*m5eC*eG*eT*eT*dG*dT*dA*dG*dT* dA*dG*dA*dT*dG*m5eC*eA*m5eC*eT* m5eC | 24 | GCGTTGTA GTAGATGC ACTC | 9 |
| 6023 -2 | 524-543 | eT*m5eC*m5eC*m5eC*eA*dG*dT*dT*dG *dG*m5dC*dG*dT*dT*dG*eT*eA*eG*eT *eA | 25 | TCCCAGTT GGCGTTGT AGTA | 10 |
| 6026 -2 | 530-549 | eT*m5eC*m5eC*eA*eG*m5dC*dT*m5dC *m5dC*m5dC*dA*dG*dT*dT*dG*eG*m5 eC*eG*eT*eT | 26 | TCCAGCTC CCAGTTGG CGTT | 11 |
| 6027 -2 | 593-612 | eT*eA*eG*eC*eA*dG*dT*dG*m5dC*dA* dG*m5dC*m5dC*dG*m5dC*eT*eT*eG*e T*m5eC | 27 | TAGCAGTG CAGCCGCT TGTC | 12 |
| 6030 -2 | 653-672 | eT*m5eC*eT*eA*eG*m5dC*m5dC*dA*d G*m5dC*dA*dG*m5dC*m5dC*m5dC*eT* eT*m5eC*eT*eT | 28 | TCTAGCCA GCAGCCCT TCTT | 13 |
| 6037 -2 | 662-681 | eT*eT*eG*eA*eA*dG*dT*m5dC*dA*dT* m5dC*dT*dA*dG*m5dC*m5eC*eA*eG*m 5eC*eA | 29 | TTGAAGTC ATCTAGCC AGCA | 14 |
| 6043 -2 | 1239-1258 | eA*eT*m5eC*m5eC*eG*dT*dG*dA*dG*d G*dG*dA*dG*m5dC*m5dC*m5eC*eT*eT *eG*eT | 30 | ATCCGTGA GGGAGCCC TTGT | 15 |
| 6049 -2 | 1379-1398 | eA*eA*eG*eT*m5eC*m5dC*m5dC*dT*d G*dT*dG*dG*dG*m5dC*dA*eA*eT*eA*e G*eA | 31 | AAGTCCCT GTGGGCAA TAGA | 16 |
| 6050 -2 | 1550-1569 | eT*m5eC*eT*m5eC*eT*m5dC*dT*dG*dG *dA*dA*dG*dT*dT*dG*eA*eT*eG*eG*m 5eC | 32 | TCTCTCTGG AAGTTGAT GGC | 17 |
| e represents a 2'-MOE sugar moiety, d represents a 2'-deoxy sugar moiety, m5[e/d]C represents a 5-methylcytosine modification combined with either an e or d modification, and * represents a phosphorothioate modification. | | | | | |

**Table 2. Nucleotide Unmodified ACVR2B siRNA**

| **Duplex** | **Position** | **SS** | **5'-3'** | **SEQ ID NO** | **AS** | **5'-3'** | **SEQ ID NO** |
|---|---|---|---|---|---|---|---|
| 6102 | 511-531 | 6102-S0 | GGGAGUGCAUC UACUACAA | 33 | 6102-A0 | UUGUAGUAGAUGC ACUCCCGU | 64 |
| 6103 | 637-657 | 6103-S0 | CCAUCGAGCUC GUGAAGAA | 34 | 6103-A0 | UUCUUCACGAGCUC GAUGGUG | 65 |
| 6104 | 658-678 | 6104-S0 | GCUGCUGGCUA GAUGACUU | 35 | 6104-A0 | AAGUCAUCUAGCCA GCAGCCC | 66 |
| 6105 | 661-681 | 6105-S0 | GCUGGCUAGAU GACUUCAA | 36 | 6105-A0 | UUGAAGUCAUCUA GCCAGCAG | 67 |
| 6106 | 672-692 | 6106-S0 | GACUUCAACUG CUACGAUA | 37 | 6106-A0 | UAUCGUAGCAGUU GAAGUCAU | 68 |
| 6107 | 736-756 | 6107-S0 | GCUGCUGUGAA GGCAACUU | 38 | 6107-A0 | AAGUUGCCUUCACA GCAGCAG | 69 |
| 6108 | 751-771 | 6108-S0 | ACUUCUGCAAC GAACGCUU | 39 | 6108-A0 | AAGCGUUCGUUGCA GAAGUUG | 70 |
| 6109 | 759-779 | 6109-S0 | AACGAACGCUU CACUCAUU | 40 | 6109-A0 | AAUGAGUGAAGCG UUCGUUGC | 71 |
| 6112 | 932-952 | 6112-S0 | CGGUCAUGUGG ACAUCCAU | 41 | 6112-A0 | AUGGAUGUCCACAU GACCGUA | 72 |
| 6113 | 1052-1072 | 6113-S0 | CCAGCUCAUGA AUGACUUU | 42 | 6113-A0 | AAAGUCAUUCAUG AGCUGGGC | 73 |
| 6115 | 1055-1075 | 6115-S0 | GCUCAUGAAUG ACUUUGUA | 43 | 6115-A0 | UACAAAGUCAUUCA UGAGCUG | 74 |
| 6116 | 1066-1086 | 6116-S0 | ACUUUGUAGCU GUCAAGAU | 44 | 6116-A0 | AUCUUGACAGCUAC AAAGUCA | 75 |
| 6117 | 1144-1164 | 6117-S0 | GCAUGAAGCAC GAGAACCU | 45 | 6117-A0 | AGGUUCUCGUGCUU CAUGCCA | 76 |
| 6119 | 1157-1177 | 6119-S0 | GAACCUGCUAC AGUUCAUU | 46 | 6119-A0 | AAUGAACUGUAGC AGGUUCUC | 77 |
| 6120 | 1192-1212 | 6120-S0 | GCUCCAACCUC GAAGUAGA | 47 | 6120-A0 | UCUACUUCGAGGUU GGAGCCU | 78 |
| 6121 | 1246-1266 | 6121-S0 | CCCUCACGGAU UACCUCAA | 48 | 6121-A0 | UUGAGGUAAUCCG UGAGGGAG | 79 |
| 6122 | 1279-1299 | 6122-S0 | CAUGGAACGAA CUGUGUCA | 49 | 6122-A0 | UGACACAGUUCGUU CCAUGUG | 80 |
| 6123 | 1283-1303 | 6123-S0 | GAACGAACUGU GUCAUGUA | 50 | 6123-A0 | UACAUGACACAGUU CGUUCCA | 81 |
| 6124 | 1286-1306 | 6124-S0 | CGAACUGUGUC AUGUAGCA | 51 | 6124-A0 | UGCUACAUGACACA GUUCGUU | 82 |
| 6125 | 1294-1314 | 6125-S0 | GUCAUGUAGCA GAGACGAU | 52 | 6125-A0 | AUCGUCUCUGCUAC AUGACAC | 83 |
| 6127 | 1298-1318 | 6127-S0 | UGUAGCAGAGA CGAUGUCA | 53 | 6127-A0 | UGACAUCGUCUCUG CUACAUG | 84 |
| 6128 | 1380-1400 | 6128-S0 | AUUGCCCACAG GGACUUUA | 54 | 6128-A0 | UAAAGUCCCUGUGG GCAAUAG | 85 |
| 6129 | 1550-1570 | 6129-S0 | CAUCAACUUCC AGAGAGAU | 55 | 6129-A0 | AUCUCUCUGGAAGU UGAUGGC | 86 |
| 6130 | 1555-1575 | 6130-S0 | ACUUCCAGAGA GAUGCCUU | 56 | 6130-A0 | AAGGCAUCUCUCUG GAAGUUG | 87 |
| 6131 | 1573-1593 | 6131-S0 | UCCUGCGCAUU GACAUGUA | 57 | 6131-A0 | UACAUGUCAAUGCG CAGGAAG | 88 |
| 6132 | 1645-1665 | 6132-S0 | GACCCGUGGAU GAGUACAU | 58 | 6132-A0 | AUGUACUCAUCCAC GGGUCCG | 89 |
| 6134 | 1667-1687 | 6134-S0 | GCCCUUUGAGG AAGAGAUU | 59 | 6134-A0 | AAUCUCUUCCUCAA AGGGCAG | 90 |
| 6135 | 1747-1767 | 6135-S0 | CCAUUAAAGAU CACUGGUU | 60 | 6135-A0 | AACCAGUGAUCUUU AAUGGUG | 91 |
| 6137 | 1750-1770 | 6137-S0 | UUAAAGAUCAC UGGUUGAA | 61 | 6137-A0 | UUCAACCAGUGAUC UUUAAUG | 92 |
| 6138 | 1919-1939 | 6138-S0 | GGUGACCUCUG UCACCAAU | 62 | 6138-A0 | AUUGGUGACAGAG GUCACCAG | 93 |
| 6140 | 1952-1972 | 6140-S0 | UAAAGAGUCAA GCAUCUAA | 63 | 6140-A0 | UUAGAUGCUUGAC UCUUUAGG | 94 |
| 6120 | 1192-1212 | 6120-S0 | GCUCCAACCUC GAAGUAGA | 47 | 6120-A0 | UCUACUUCGAGGUU GGAGCCU | 78 |

Human ACVR2B, mRNA (SEQ ID NO. 1, NCBI Accession No. NM_001106.4)

1 ggctcggctc cgtggccgcc gctcaggagc cattttggac tcggttcagc tcccctcccc
61 cccacccctc cccccgttca tggcccctcc ggactcggcc cctgcgcccg gggcccgggc
121 ccagccccgc cgcgctatgc ctgagtcggg cgcgccccgg cccgtgcccc gccgccgccc
181 cccggccccc gcgtcgcccc ggagcccggg ccgcagcctg cgccgcccgc agcggccctg
241 agcccggccc cgccgaccgg cccttggagc ccgaacgctg ctcggggacg aaggcgcagg
301 aagcgcgcag ggaacgagac cgaaggaagg agcgggaagg agagcgcagc cgccgcctgg
361 ccctgcgcgc cccgggagcg ccgtgcggcc ctgcccgcgg gctccgggtg tgcgcggggc
421 ggcgccgcgg aacatgacgg cgccctgggt ggccctcgcc ctcctctggg gatcgctgtg
481 cgccggctct gggcgtgggg aggctgagac acgggagtgc atctactaca acgccaactg
541 ggagctggag cgcaccaacc agagcggcct ggagcgctgc gaaggcgagc aggacaagcg
601 gctgcactgc tacgcctcct ggcgcaacag ctctggcacc atcgagctcg tgaagaaggg
661 ctgctggcta gatgacttca actgctacga taggcaggag tgtgtggcca ctgaggagaa
721 cccccaggtg tacttctgct gctgtgaagg caacttctgc aacgaacgct tcactcattt
781 gccagaggct gggggcccgg aagtcacgta cgagccaccc ccgacagccc ccaccctgct
841 cacggtgctg gcctactcac tgctgcccat cgggggcctt tccctcatcg tcctgctggc
901 cttttggatg taccggcatc gcaagccccc ctacggtcat gtggacatcc atgaggaccc
961 tgggcctcca ccaccatccc ctctggtggg cctgaagcca ctgcagctgc tggagatcaa
1021 ggctcggggg cgctttggct gtgtctggaa ggcccagctc atgaatgact ttgtagctgt
1081 caagatcttc ccactccagg acaagcagtc gtggcagagt gaacgggaga tcttcagcac
1141 acctggcatg aagcacgaga acctgctaca gttcattgct gccgagaagc gaggctccaa
1201 cctcgaagta gagctgtggc tcatcacggc cttccatgac aagggctccc tcacggatta
1261 cctcaagggg aacatcatca catggaacga actgtgtcat gtagcagaga cgatgtcacg
1321 aggcctctca tacctgcatg aggatgtgcc ctggtgccgt ggcgagggcc acaagccgtc
1381 tattgcccac agggacttta aaagtaagaa tgtattgctg aagagcgacc tcacagccgt
1441 gctggctgac tttggcttgg ctgttcgatt tgagccaggg aaacctccag gggacaccca
1501 cggacaggta ggcacgagac ggtacatggc tcctgaggtg ctcgagggag ccatcaactt
1561 ccagagagat gccttcctgc gcattgacat gtatgccatg gggttggtgc tgtgggagct

1621 tgtgtctcgc tgcaaggctg cagacggacc cgtggatgag tacatgctgc cctttgagga
1681 agagattggc cagcaccctt cgttggagga gctgcaggag gtggtggtgc acaagaagat
1741 gaggcccacc attaaagatc actggttgaa acacccgggc ctggcccagc tttgtgtgac
1801 catcgaggag tgctgggacc atgatgcaga ggctcgcttg tccgcgggct gtgtggagga
1861 gcgggtgtcc ctgattcgga ggtcggtcaa cggcactacc tcggactgtc tcgtttccct
1921 ggtgacctct gtcaccaatg tggacctgcc ccctaaagag tcaagcatct aagcccagga
1981 catgagtgtc tgtccagact cagtggatct gaagaaaaaa ggaaaaaaag ttgtgttttg
2041 ttttggaaat cccataaaac caacaaacac ataaaatgca gctgctattt taccttgact
2101 ttttattatt attattataa ttattataat tattattatt aatattattt tttggattgg
2161 atcagttttt accagcatat tgctctactg tatcacaaac agcggacacg tcagcaggcg
2221 ttgaggtgct gagctgtgga tgcagaacca gcgccatgct gaagagcctc agccacctcc
2281 tgtcctttgg gattcgtttt tcccgctttc tctttgtttg tcgtctcaga atctgtgaca
2341 caaagaaacc catctcctgt cttaggaaac ctaatgctgc aaactctacc tagaggaacc
2401 tttgaagact gttacataag aacatacctt cctcagaaga ggagtttcct ctgccctctg
2461 cccttctccc ctgcctccct ccctcccctc cttttatttt gttttagtga gcttaagaaa
2521 cagcagatgt gtctttcacg gatctaacgg gtgttgtcct gatcgagaaa aaaactggga
2581 tgagaatggt ttggactgga gttggaaggg gaggacggta ctgggggtag ggtttggaac
2641 agagctacac tggactcggg cacattcgga gcagcatcct ttagtatgga ggctacttct
2701 caggtaacca ggaattgagg ggaaggacct tgtggaggcc gagcattaac agcaagagcg
2761 gggtttggag aaagtctgag attgggtgca gccctgactt acctgctggc cctgaccagt
2821 ttcttttcac taacttggcc ttgggcatag gatgaaacat tttttctgcc ctaattttaa
2881 aactaggtga gggtagaatc atcacaggtt aggaatacat tcttcataag acacgatgct
2941 gtaaataccc ttaatggacg aaaagttgaa atacttttgt ttcctcttgg agcagttcag
3001 ggaaatgccc acaggggatt gtcctgcaca gatagggcaa gaggatttcc tgggtggagt
3061 ctgccaaggc ctgcctcgct ggggacccca gagtcctgca cctctggttc cgccccaggt
3121 ggtgacatta ctgtccccgt tctgtggctc gtggacaaga ctttctccag accccttaaa
3181 gtggtacata ttctaaaaaa ctgtttttct attatgccat aaccttgctc tagtcagtga
3241 atgttcctaa tgctgctgtt tcaacatttg aattcttttt aatttatgaa acatgctaaa
3301 tttttttttt caaacaaaac acacacatcc acatatacac atgcttcgct atgtggcttc
3361 caaggtttaa attttgaaaa gtaaaagaat taaaacttca cgaccacaga tcacctcaaa
3421 ccagaaatac ctcagaattt tctacttatg taaggtttat tatatatttt gttagttgtg
3481 ttgtcttgta gtaagtatat tttaatgtaa gttggctttt gtgacaagga agtttaaaag
3541 aaatagagaa aaagaaaaaa gtttgcatct tctagggagt gctaccattt ttgtttgata
3601 acgccccctt gtaaataatt gtcatcaact gtaggttggc tgtctgggcc aagtctgggc
3661 atttatcagt cttgtttgtg aaggcttttc cttctggttt ctttagatca ttttatttaa
3721 aaacagtgca tctcttcatc gtgagggtag gcaaggcggg ggccgtgggg agaggttgac
3781 ctgggtgaga actgaagagg ccgcctcctc ttgggttgtt tggagcttca catgtaattc
3841 acatgtaaca tgtaacttga tcggtcagtg ttcagaatga caagtaaccc cgcttaaact
3901 tggtagaagg atggccctta gacctgaatg gggtgatttt acttgggatt taacttcttc
3961 agcaaattaa cagcaacgtt ggaagagatc tgtggcgcct ctgtgaagca caccgtgact
4021 caggccagtc ttttagtgca gcgtgtctgg gagtgaaggg ttttgccctt gctggtcttg
4081 gagtccacag tgtgaggggc actgcacatg cctgggcatc tacctagtgt gctatgttca
4141 gtgtctgggg cttactgccc cggggtcctt tcctctgggt gttggggcac agggtgctat
4201 gggaggccca tttgcttccc tctcggagct cagtttttgc ttcatgggtc aaaatgtggg
4261 ctggccaagt ggttacagga acagggtttc ggtaagctat gttgtctttt tttttttttt
4321 tttttttttt tttaatggtt tgattttgtg ctgtggtatt ttttttccct tagaataatt
4381 tttaatggca aaacaggcct tacagcagtt gcttttcttt accatttatt tctttaagaa
4441 gctttaaaat atttattgaa aagtgccata tctaatttct ttagctttcg cctcaggcag
4501 tgcaggcatc tttacttttc atcctcagaa gaaacaaacg actaacaaat gtagcaaatt
4561 tactgcagga atagttaggt catgatacta cctgaacact aaaccccagc ctctttgttt
4621 ggttttagtt cctctgggtg gtttttcttt tgtgtgctgg cttgattctt gtgagaagtt
4681 ttgacctggc caagggaggg ttgagccatg gttctggtgt gggactttgc ggtcaagaca

4741 cagtacagac aggtcaggcc tgcgtgcctt ttctctgggt ggcctccccg ttaggcccac
4801 cgtacgctca gccactatag tgtccctgtg gggccttgcc atcagattgt gtgtcaggag
4861 atggtacctt tttggtgtgg ctggggagga gtgtggtcca tgccagttct ttgggcttca
4921 ggccactctt cccctcatgc tgtggtgtaa agtgcaccca tcaggtggta tatctggttc
4981 tgatggcaag aagaaggtgg gggatctcct tatagggcat gggtctagga gcacagatgg
5041 gccttttgcc ccgggtaaat gcttgtctgt ttgctgtcat gtgttctttg aggagtgagc
5101 catctcgagc cctgctttga atttactggg tcatagagcc tctgcctgtg ctcttttcca
5161 taatgacttc atgtgacatg cacttttggt gggctcagat aattggtttc tttttgtttt
5221 tgacctcagg ctctgtggca gactggggaa aatggggcct ggcatcattt tccctgtcaa
5281 tgggaggggc tgttccatgc agggtgggag gggaccaagt tagcagagag tagccaagga
5341 tccttgcttc ttcctttcta gtgtgctgtc atccaagcag gctcctggct gtagggatgg
5401 gccttgggga agaatcttct ttgaaagcat ctatgataac tgagaagtca tccctagttg
5461 gagaaatcca gtaatgagca gaaggaggaa gcaagtgagg acagaggcca ttgtattaca
5521 gtgtcacgca gagggccctc aatgatgggg cattggggaa ggctgtagac atagtcatca
5581 gaacatcctg gcctggcata agctgggttt tctcctggga ccattggtcc tcagcaggag
5641 ttctttgcat gagttgctca ggggcaaggg ctgcaagtgg gctgtgctta ggagaaagtg
5701 acacctggca gtgagggaag atggtgagca ttattagcct ttgttgtcca gcatggcctt
5761 cttgtcctgt ctgctctgga gaggagcctg tgggaccagt cctgcctggg gagggcatac
5821 ccacacgtgc cagctgattc tgactctgaa tacatcatgt ccggacttgg ggtgtttct
5881 gcagaaaaag gaggttgttt ttcagccttg aacatcttca ggaggataga gactcttgct
5941 cacatattct tagcaaaggg aagggtctct catctccagg ccacagagat agttcttcca
6001 ttgccctaag aggctaggct aaccctcttg acataactta gacagcaaag cacttcatcc
6061 tgtagttggg ctctgtcacc tttctcttca gttggccaca ttctcgtttc ctccatcctg
6121 ctatgctttg tgtgctcggg ctgtgtgtgg ggtttttccc tggtggaagg aagcccagct
6181 gtgtattgaa tgtccttcat gtgttgtgtg tggctcagaa agcctgtcac ttggcccctg
6241 tgctctgagc cgtgagggtg gggaggtggc tgttccatta aagtgggagt attggatggc
6301 cctcttgaaa ctagaatttt gcctttttta gtatgcagta taaagtttcc agcatctatt
6361 ggtaacacaa agatttgctg gtttttaaaa taatacagta agcataagta tgtaagtttt
6421 tagaattggt actagaagtt ggacagctag ttattctcga gaactttatt tcactagaaa
6481 aatatactaa ttggaaagca gtttccagga gttaactcag tttaattttc agtctcagtt
6541 attttagcct gttgagtttt tgatggcaca cctttggaga gatggccacg cctgattccc
6601 atttcagggg catcagacca taccttttta agaagctccg tgaatctagt catctaccct
6661 tcatcctggg cgaacagcca aaaagagaag gggacaaggt gtctttttct ccttctcact
6721 ggggtgacat gaattctttt agttaatggc tgtttgcaaa ttctaaacta atgaaatact
6781 tagcagctaa catgttcaat ctagtaatga tgagtttaaa tctcaattga cagtaatgtt
6841 ttagataaac aggcccagta attcagttga tgaactgtat atcttctcag tctagatttg
6901 taaatgttta atgaattcag ggttataagc atagttcttt aagtaagatt ccagatagtt
6961 gatttgcaac cagcagtcta cctatgaatg tatcccaaac ctttagaaga ttggaaaaga
7021 tttttgaaat aatgatttag ttttgtagga aaaacacccc cttgaaaatt aattcggttg
7081 acccagtaac attttttaaa acaattggtg gctccaaaag gcctgccaac aaagaaaagt
7141 ccaaattatc tagtgggaca ttttgaatgt tttatgttta ttttgggtcc actgtaaact
7201 ttggttcaaa aaagaatttg aatttaaaga atttaccatt atttaaatta ttaccaagtt
7261 tttacatttt catgatggta ttttccaggt atgaatgaaa catgactttt tgattgtggt
7321 acttcctgta tcccctgtag tgccaaaacc agtgatactt tatttgctcc tatggcagct
7381 catagaggta accgaagtga tttttcctca gtaattgaaa cacatattct ctaaatgcca
7441 atgtgtggtg atgggccctg cactgccttc atttctctag ggcagtgtct ttggattgtc
7501 tagggcctag gtaattctga gaactactgt aaaccaacca cagggcacta aagcaatgta
7561 cacaccactc tttgtgtgta tggaaggggt tatataaacc tgggctatgc tggacatcta
7621 cagaagagta ttacattcac ttgcaaagtt tacatttttg agctcacagt tatgaaaaat
7681 atgacccaca agtttttcag gcaggtgagg atgggtcttc ttgcaaatgc atgagttctg
7741 tcttgagtcc tgggaacttc tctgttggtt gagtgtgggc tcattccctg actctcctaa
7801 tcatgtttgc gtcagaatgt tagcattgta aataaaagaa taggttgtat aatagataca

7861 caacacttga aactttactt taaaaaaatc gatagttcta catatatatt tagttatatc
7921 acttgacaga tttcttctac acagtgtgga gattgtttta taccacagat tatttttata
7981 aagttagtga atttgaatga ttttgtaatc agagctaatg agctttacct ttcaagagaa
8041 acgtacactg gagcatgagt ggtgtggaac ttttacttag tgtttatatg gattcttgtg
8101 atacactggc agactggagt caatttgcgg gtcttttttg gccaaaactc cacttgtggt
8161 tgtgtaggac agtgatattc agctcagctt cttgtggatt gggaggagag agggcctgca
8221 atgtgtttta cattggtgct tcctcctgag atttctgttg aacaaagggt tctgaggtca
8281 aaaattagtt tgtaagcctt tgccatagga catagtcatg tgagagtgtt tggggaaca
8341 gaaattgtat aggggtgcct attggggtgg gatgggactc gaataagatt caggtacaaa
8401 aactttgaaa tgagaatctg gtggtttgag taatccacca gactgaatta tctaagatca
8461 cattatccag gttggggggc agaattaccc agttaagtaa ttgttcagaa aagtggggag
8521 ggtggcatgt ggatgcagtg atccaattaa atggagagct gccaggcaca ttttgtcctc
8581 tctggtcagt gagaatggtt gggttggctc gctgcttcaa tctgtggaat cagccaggag
8641 cccagtgagg aagctcagaa ccccagtaac agcagagcat ctttcagata gctccagagt
8701 tttcctgctt ttctgaggaa gctcagcatc actgccacaa tacggaaagt ggtcttcatt
8761 ttagcctatt tatttttagg cagagagtgg atggttattt gtgtgggact tttggtggcg
8821 atatataatg aataattaag ttaatttctg gtatgcataa tggccagtcc tgaggcccag
8881 ctgaagacct gtcccccaga ccctgcccgc tggcttcagg ctgctgcttc tagacagagg
8941 tgcactggac gggatagttt tatcaagaga atccctaatg tgtcatttta aaccagctgt
9001 gctttttatt cattctggtt gagcgtatag gtttacactt tacccttttt atacttggaa
9061 taaatttagt tccagcagat ctagtagcac tccagaaacc aaccccatct gttccccata
9121 aaaagaacat tttctctgct ctccagccac gtgtcttgga atgtaattct gttgtgcctt
9181 tgtttttatc actctcttcg ccccaaaagc aactgctgta agcttttttc tacttgtctt
9241 ttctagtccc caacctctac ctttttcctt tttcccagcc ctaatttctg gatgcacttc
9301 tgtgatccag gtattttaag aaccagttac ctcagacctc atgttgaaca gtgtcgccat
9361 ctgggtcctc ttgatactgc agacttttaa cgtacacatg caggaaccct gctgagcgtg
9421 ggcacttgtt ttaaagcaaa actcttccca aggactgaag aaagggcttc tggcaagctc
9481 gtcatggcat tgtggtggga tgggtctaga gtgtcatctg aatggtgctt cctgtgttcc
9541 tctttgaatt ctgccatttt cagtattctt gtgtgtctga ataggcaaag cgatttaatt
9601 ggctggtctt gcacgcaaat tagttccaaa gataagctct ttgtaacaca tttccagtcg
9661 ctaatgctca aatgtagaac attcctttaa atggcaggat aaaaaaccca ctatccacca
9721 tagtgcattt tgggaagatg tctgtagcat atgttgctgt gaaattaggc cttgtgggat
9781 atggctgttt gtcattttga tgtattttaa ataaatatat atatttttta aagagccttt
9841 tttaccagtt caaaaagttt aattaaccag cagtcaccgc atctgaattt ttgtctctgg
9901 ggcatagatg gcagaccaag attaaaagtg gtaactcagc tatacgagca tgggctacct
9961 tcctgggctc tcctgcagtc ctgtagacct gctgttccgc agaccatggg acacaaggtc
10021 agtgtgttcc cagtgagggt cccaagtcag tcatcttaag tgtttgttct ctgccccatt
10081 cagtggactg ttgacttcag tccctgcaag tgctttagcc cgagtggggt tttctcagag
10141 cactgccacg agttaagtgt gtgtttagcc aaataatttc tccgtaaggg aaaaatgcag
10201 tcacccaaat tttaccaaca atgacagaga tgagagtaga aaagattagg caacatctga
10261 gttttaactt gaaaagtgtc caagtcatca tgaaaggccg actgggagca agtgattatt
10321 agagattctt caggagacct catctgaaaa tgttaagact gccagtgagg gaaggaattg
10381 ttaaaatgcc agcggctttt ttttcctctt tttttctgta attctgtaaa aatgcagaga
10441 aagttgagtg gtacttcaga attgagggag agggttaccg cagagtagaa atatatttct
10501 agatttcagt tccacaccac aaatccacaa caatgccatt tttcaactgt acaaaaatct
10561 gcttatgaac tggacatgat cttaatggta gtgtcaaagg ccaagttttt cacctgttaa
10621 tatttttcca catttgtcct tgaatctgaa taactttata cagtactgta aatttaactt
10681 acatcgagtt tgttgtcaat tcttatgaaa agagctttct gcatgtaaca catacggtta
10741 aagaacacag caaaggacaa aatttgcagg aacagttttg gaaccaacag aaaatgtcac
10801 cttttatttg ccatcttata tatatctatc agttttacca gctacttcta aatttgtaca
10861 ttatttgtaa gggaaagaag gaaaacccta agacttgtct aacttagtgg agaatgtgtg
10921 tgttgggctt aggatggata gctaagtctt attgagctgt gttacctaac ttgtatataa

10981 aaattgtaat taaaagtttg ggttcacctg tttctcacag tttaaaatga tgagtaattg
11041 caaactctgg aaatgtgact agtatatgat ttaaggctgt agaagcaagg aagctctttc
11101 aagtgctaaa actaaagact tctagttttt ggctcaaata agtactgttt gtataccagg
11161 atatgtgaga tgtaaatgta gtaggtcact tttcaccctt gtagctataa aataaaaatt
11221 ttgtagaaca gaaatagctt gtactactga attaacaaaa gttatactaa agtatcatgt
11281 ttaaaaaaaa tatatatata tatacagagt taagcttgtt gctgttaccc tgtctggatt
11341 tgaaaagtgt gctgatttat atatatatat tacacacaca cacacacaca cacacacaca
11401 cacacacaca cacacacaca cacacacaca cacacacaca cacatacacc taaaatggcc
11461 taaagcagac atccatgtaa ttacagttgc aaaatgaaaa cattttggaa agaacattgt
11521 atcatagttc attcatttgc agtggatctt tgttcctttt tactgtggta attttagaaa
11581 tgagtgtcaa gtttgaaatt agatctgcta agttggggtt ttgctgcttg aactctgcac
11641 tgggtcctca aataaaccga tgtgaatgta gttttttccc cctgtgtgaa gaagcagtta
11701 caccccaaca ataggaggaa aaatctagaa ctatttcaag ttttatcttt ttgtatatga
11761 aaataaaata ataataaaac aa

Cynomolgus Monkey ACVR2B mRNA (SEQ ID NO. 2, NCBI Accession No. XM_015445674.2)

1 gcgtcgcccc ggagcccggg ccgcagcctg cgccgcccgc agcggccctg agcccggccc
61 cgccgaccgg cccttggagc ccgaacgctg ctcggggacg aaggcgcagg aagcgcgcac
121 ggaacgagac cgaaggaagg agcgggaagg agagcgcagc cgccgctggg ccctgcgcgc
181 cccgggagcg ccgtgcggcc ctgcccgcgg gctccgggtg tgcgcggggc ggcgcagcgg
241 aacatgacgg cgccctgggc ggccctcgcc ctcctctggg gatcgctgtg cgccggtaag
301 aactgggcgc ggcgcgggta cgcagagagg gcgcgcaggg ctggcccctg tcgccgcgcg
361 gtgtttacca acaaagggcg ggcccgcggc ctcgtcgccg cgccacctgt attcagctcg
421 catctcccgg ggcgggggtt gggggcgcgg tgggcgccca gctcgccgat cttggggaca
481 cgatcttgtt tgtgcagtcg tctgcagagt tctggttcct agaggtgccc ctgcgatggg
541 ggccgggacc cggagcctca gggtcggagt tcgggctacg cgggcgcgtg gaacccaggg
601 aaagagctct gggcgtgggg aggctgagac acgggagtgc atctactaca acgccaactg
661 ggagctggag cgcaccaacc agagcggcct ggagcgctgc gagggcgagc aggacaagcg
721 gctgcactgc tacgcctcct ggcgcaacag ctctggcacc atcgagctcg tgaagaaggg
781 ctgctggcta gatgacttca actgctatga taggcaggag tgtgtggcca ctgaggagaa
841 cccccaggtg tacttctgct gctgtgaagg caacttctgc aacgagcgct tcactcattt
901 gccagaggct gggggcccgg aagtcacgta tgagccacct ccgacagccc ccaccctgct
961 cacggtgctg gcctactcac tgctgcccat tgggggcctt tccctcatcg tcctgctggc
1021 cttttggatg taccggcatc gcaagccccc gtacggtcat gtggacatcc atgaggaccc
1081 tgggcctcca cccccatccc ctctggtggg cctgaagcca ctgcagctgc tggagatcaa
1141 ggctcggggg cgctttggct gtgtctggaa ggcccagctc atgaatgact ttgtagctgt
1201 caagatcttc ccactccagg acaagcagtc gtggcagagt gaacgggaga tcttcagcac
1261 acctggcatg aagcacgaga acctgctaca gttcattgct gccgagaagc gaggctccaa
1321 cctcgaagta gagctgtggc tcatcacggc cttccacgac aagggctccc tcacggatta
1381 cctcaagggg aacatcatca catggaacga actgtgtcat gtagcagaga cgatgtcgcg
1441 aggcctctca tacctgcatg aggacgtgcc ctggtgccgt ggcgagggcc acaagccgtc
1501 tattgcccac agggacttta aaagtaagaa tgtattgctg aagagcgacc tcacagccgt
1561 gctggctgac tttggcttgg ctgttcgatt tgagccaggg aaacctccag gggacaccca
1621 cggacaggta ggcacgagac ggtacatggc tcctgaggtg ctcgagggag ccatcaactt
1681 ccagagagat gccttcctgc gcattgacat gtatgccatg gggctggtgc tgtgggagct
1741 tgtgtctcgc tgcaaggctg cagacggacc cgtggatgag tacatgctgc cctttgagga
1801 agagattggc cagcaccctt cgttggagga gctgcaggag gtggtggtgc acaagaagat
1861 gaggcccacc attaaggatc actggttgaa acacccgggc ctggcccagc tttgtgtgac
1921 catcgaggag tgctgggacc atgatgcaga ggctcgcttg tctgcgggct gtgtggagga
1981 gcgggtgtcc ctgattcgga ggtcggtcaa cggcactacc tcggactgtc tcgtttccct
2041 ggtgacctct gtcaccaatg tggacctgcc ccctaaagag tcaagcatct aagcccagga
2101 catgagtgtc tgtccagact cagtggatct gaagaaaaaa ggaaaaaaag ttgtgttttg

2161 ttttggaaat cccataaaac caacaaacac ataaaatgca gctgctattt taccttgact
2221 tattattatt attataatta ttataattat tattattaat attatttttt ggattggatc
2281 agtttttacc agcatattgc tctactgtat cacaaacagc ggacacgtca gcaggcgttg
2341 aggtgctgag ctgtgaatgc agaaccagcg ccatgctgaa gagcctcagc cacctcctgt
2401 cctttgggat tcgtttttcc cgctttctct ttgtttgtcg tctcagaatc tgtgacacaa
2461 agaaacccat ctcctgtctt aggaaaccta atgctgcaaa ctctacctag aggaaccttt
2521 gaagactgtt acataagaac ataccttcct cagaagagga gtttcctctg ccctctgccc
2581 ttctcccctc cttccctccc tccctccctc ccttttattt tgttttagtg agcttaagaa
2641 acagcagata catctttcac ggatctaacg ggtgttgtcc tgaccgagaa aaaaactggg
2701 atgagaatgg tttggactgg agttggaagg ggaggacagt actgggggta ggatttggaa
2761 cagagctaca ctggactcgg gcacattcgg agcagcatcc tttactatgg aggctacttc
2821 tcaggtaacc aggaatcgag gggaaggacc ttgtggaggc cgagcattaa tagcaagagt
2881 ggggtttgga gaaagtctga gattgggtac agccctgact tacctgttgg ccctgaccag
2941 tttcttttca ctaacttggc cttgggcata gggtgaaaca ttttttcttc cctaatttta
3001 aaattaggtg agggtagaat cacaggttag tgaatacatt cttcataaga cacgatgctg
3061 taaataccct taatggacga aaagttgaaa tacttttgtt tcctcttgga gcagttcagg
3121 gaaatgccca cagggggttg tcttgcacag atagggcaag aggatttcct gggtggagtc
3181 tgccaaggcc tgcctcgctg gggacccaga gtcctgcagc tctggttctg ccccaggtgg
3241 taacaatact gtcccctttc tgtggctcgt ggacaagact ttctccagac cccttaaagt
3301 ggtacatatt ctaaaaaaac tgtttttcta ttatgccata accttgctct agtcagtgaa
3361 tgttcctaat gctgctgttt caacatttga attcttttta atttatgaaa catgctaaat
3421 ttttttttca aacaaaacac atacatccac atatacacat gctttgctat gtggcttcca
3481 aggtttaaat tttgaaaagt aaaagaatta aaacttcacg accacagatc acctcaaacc
3541 agaaatacct cagaattttc tacttgtgta aggtttatta tatattttgt tagttgtgtt
3601 gtcttgtagt aagtatattt taatgtaagt tggcttttat gacaagaaag tttaaaagaa
3661 atagagaaaa agaaaaaagt ttgcatcttc tagggagtgc taccattttt gtttgataac
3721 gcccccttgt aaataattgt catcagctgt aggttggctg tctgggccaa gtctgggcat
3781 tcatcagtct tgtttgtgaa ggcttttcct tctggtttct ttagatcatt ttatttaaaa
3841 acagtgcatc tcttcatcgt gagggtaggc aaggcggggg ctgtggggag aggttgacct
3901 gggtgagaac tgaagagtcc gcctcctctt gggttgtttg gagcttcaca tggaattcac
3961 atgtaacatg taacttgatc cgtcgatgtt cagaatgaca accccactta aacttggtag
4021 aaggatggcc cttagacctg aatggggtga ttttacttga gatttaactt cttcagcaaa
4081 ttagcagcaa cgttggaaga gatctgtggc acctccgtga agcacaccat gactcaggcc
4141 agtcttctag tgtagcgtgt ctggagggaa gggtttcgct cttgctggtc ttggagtcca
4201 cagtgtgggg gggcactgca cacgcctggc catctacctg gcgtgctatg ttcagtgtct
4261 ggggcttgct gcccgggggt cttttcctct gggtgttgag gcacagggtg ctatgggagg
4321 cccgtttgct tccctctcag agctcagttt ttgcttcatg ggtcaaaatg tgggctggcc
4381 aagtggttac aggaacaggg ttttggtaag ctgtgttgtc tttttttaat ttttattttt
4441 aaatggtttg attttgtgct gtggtatttt tcttcccttg gaataatttt taatggcaaa
4501 acaggcctta cagcagttgc ttttctttac catttatttc tttaagaagc tttaaaatat
4561 ttattgaaaa gtgccatatc taattttttt agctttctcc tcaggcaggg caggcatctt
4621 tacttttcat cctcagaaga aacaaaccac taacaaatgt agcaaattta ctgcaggaat
4681 agttagatca tgatactacc tgaacactaa accccagcct ctttgtttcg ttttagttcc
4741 tctgggtggt ttttcttttg tgtgctggct tgattcttgt gagaagtttt gacctggcca
4801 agggagggtt gagccatggt tctggtgtgg gactttgcag tcaggacaca gtacagacag
4861 gtcaggcctg cctgcctttt ctctgggtgg cctccccctt cggcccactg tgtgctcagc
4921 cactgtagtg tccctgtggg gcctcgccat cagatggtgc gtcaggagat ggtacgtttt
4981 tggtggggct ggggaggagg gtggtccatg ccagttcttt gggcttcagg ccgctcttcc
5041 cctcatattg tggtgtaaag ctcacccatc aggtggtata tctggttctg atggcaagaa
5101 aaaagtgggg gatctcctta tagggcatgg gtctaggagc acaggtgggc ctttgcccc
5161 aggtaaaatg tttgtctgtt tgctgtcatg tgttctttga ggagtgagcc atctagagcc
5221 ctactttgaa tttactgggt catacagcct ctgcctgtgc tgttttctgt aataacttca

5281 tgcaacatgc acttttcggg ggctcagata attgctttct ttttgttttt gacctcaggc
5341 tctttgacag actggggaaa atggggcctg gcatcgtttt ccctgtcaat gggaggggct
5401 gttccatgca gggtgggagg ggaccaagtt agcagagagt agccgaggat ccttgcttct
5461 tcgtttctag tgtgctgtca tccaagcagg ctcctggctg tagggatggg ccgtggggaa
5521 caatcttctt tgaaagcatc tgtgataact gagaagtcat ccctagctgg agaaatccag
5581 taatgagcag aaggaggaag caagtgagga cagaggccat tgtgttacag tgtcatgcag
5641 agggccctca gtgatggggc actggggaag gccatagaca tagtcatcag aacatcctgg
5701 cctggcataa gctggatttt ctcctgggac cattggtcct caacaggagt tccttgcatg
5761 agttgcttgt gggcaagggc tgccaatggg ctattcttag gagaaagtga caccggcctg
5821 tgagggaaga tggtgagcat tattaggctt tgtgtccagc atggccttct tgtcctgtct
5881 gctctggaga gaagcctgtg ggaccagtcc tgcctgggga ggggggaggg catatccaca
5941 catgccggct gattctgact ctgaatacat cctgtctgga cttgggggtg tttctgcaga
6001 acaaagaggt tgttttccag ccttgaacat cttcaggagg atagagactc ttgctcacat
6061 attcttagca aagggaaggg tctctcttct ccaagccaca gagatagttc ttcgattgcc
6121 ctaagaggct aggctgaccc tcttgacatg acttagacag caaagcactt cgtcctgtag
6181 ttgggctctg tcacctttct ctttagttgg ccatattctc gtttcctcca tcctgctact
6241 atgccctgtg agctctggct gtgtgtgggg tttttccctg gtggaaggag gcccagctat
6301 gtattgaatg tccttcgtgt gttgtgtgtg actcagaaag cctgtcactt ggcccctgtg
6361 ctctgagcca tgagggtggg gaggtggctg ttctattaaa gtgggagtat tggatggccc
6421 tcttgaaact agaattttgc cttttttagt atgtagtata aagtttccag cacctattgg
6481 taacacaaag acttgctggt ttttaaaata atctaataag cataagtatg tacgttttta
6541 agtttttaga attggtacta gaagttggac agctagttat tattctcgag aacttgattt
6601 cactagaaaa ataaactaat tggaaagcag tttccaggag ttaactcagt ttgattttca
6661 gtctcagtta ttttagcctg ttgagttttt gatggcacac ctttggagag atggccaggc
6721 ctgattccca tttcaggggc atcagaccat acttttttaa gaagctccgt gaatctagtt
6781 atctaccctg catcctgggc aaacagccag aatgagaagg ggacaaggtg tctttttctc
6841 cttttcactg gggtgacatg aattctttta gttaatggct gtttgcaaat tctaaactaa
6901 tgaaatactc agcagctaac atgttcaatc tagtaatgat tagtttaaat ctcaattgac
6961 agtaatgttt tagataatgt tttagataaa caggcccagt aattcagttg atgaactgta
7021 tatacatctt ctcagtttag ttttgtaaat gtttaatgaa ttcagggtta tacacataat
7081 tcttttaagt aagattccag gtagttgatt tgtaaccagc agtctaccta tgaatgtatc
7141 ccaaaccttt agaaggttgg aaaagatttt tgaaataatg atttagtttt gtagaaaaaa
7201 cacccctttg gaaattaatt cagttgaccc aataacattt tttaaaacaa ttggtggctc
7261 caaaaggcct gccaacaaac aaaagtccaa attatctagt gggacacttt gaatgtttta
7321 tgtttatttt gggtccactg taaactttgg ttcgaacaag aatttgaatt taaagaattt
7381 atcattattt aaattcttac caagttttta cattttcatg acggtatctt ccaggtatga
7441 atgaaacaag acttttttta ttgtggtact tcccgtatcc cctgtagtgc caaaaccagt
7501 gatactttat ttgctcctgc ggcagctcat agagataact gaagtgattt ttcctcagta
7561 attgaaacac atattctcta aatgccaatg tgtggtgatg ggccctgcat tgcctttatt
7621 tctctagggc agtgtttgga ttgtctaggg cctaggtaat tctgagaact actgtaaacc
7681 aaccacagga cactaaagca atgtacacac cactctgtgt gtgcacgtaa tgggttatat
7741 aaacctgggc tatgctggac atctacagaa gagtattaca ttcacttgga agtttacat
7801 ttttgagctc acagttacga aaaatatgac ccgcaagttt ttcaggtagg tgaggatggg
7861 tcttcttgca aatgcatgag ttctgtcttg agtcctggga acttctctgt tggttgggtg
7921 tgggctcatt ccctgactgt cctaatcacg tttgcgtcag aatgttagca ttgtaaataa
7981 aagaataggt tgtataatag atacacaaca cttgaaactt tactttaaaa aaatcgatag
8041 ttctacatat atatttagtt atatcacttg acagatttct tctacacagt gtggagattg
8101 ttttatacca cagattattt ttataaagtt agtgaatttg aatgattttg taatcagagc
8161 taatgagctt tacctttcaa gagaaacgta cactggagca tgagtggtgt ggaactttta
8221 cttagcgctt atatggattc ttgtgataca ctggcagact ggagtcaatt tgcgggtctt
8281 ttttggccaa aactccactt gtatttgtgt aggacagtga tacttcagct cagcttcttg
8341 tagactggga ggagagaggg cctgcagtgt gttttacatt ggtgcttcct cctgagattt

8401 ctgttgaaca aagggttctg cggtcaaaaa ttggtttgta agcttttgct ataggacata
8461 gtcatgtgag agtgtttggg ggaacagaaa ttgtataggg gtgcagattg gggtgggatg
8521 ggactcgaat aagattcagg tacaaaaact ttgaaatgag aatctggtgg tttgagtaat
8581 ccaccaaact gaattaccta agatcacatt atccaggttg ggggacagaa ttacccagtt
8641 aagtaattgt tcagaaaagt ggggagcgtg gcatgtggat gcagtgatcc aattaaatgg
8701 agagctgcca ggcacatttt gtcctctctg gtcagggctc ttttggaggt gagaatggtt
8761 gggttggctc gctgcttcaa tctgtggagt cagccaggag cccagtgaag aagctcggaa
8821 ccccagtaac agcagagcat ctttcaaata gctccagagt tttcctgctt ttcttaggaa
8881 gcttagcatc actgccacaa tatggaaagt ggtcttcatt ttagcctgtt tatttttagg
8941 cagagagtgg atggttattt gtgtgggact tttgatggcg atctataatg aataattaat
9001 ttctggtatg cataatggcc agtcctgagg cccagctaaa gacctgtccc ccagaccctg
9061 cccgctggct tcaagctgct gcttccagac agaggtgcac tggactggat agttttatca
9121 agagaatccc taatgtgtca ttttaaacca gctgtgctta ttattcattc tggttaagcg
9181 tataggttta cactttaccc tttttatact tggaataaat ttagttccag cagatctagt
9241 agcactccag aaaccaaccc catctgttcc ccataaaaag aacatttctc tgctctgcag
9301 ccacgtgtct tggaatgtaa ttctgttgtg cctttgtttt tgtcactctc ttccccccaa
9361 aagcaactgc tgtaagcttt tttctacttg tcttttctag tcccccacct ctaccttttt
9421 cctttttccc agctctaatt tctggatgca cttttgtgat ccagatattt taagaaccag
9481 ttacctcaga cctcatgttg aacagtgtca ccatctgggt cctcttgata ctgcagactt
9541 ttaacgtaca catgcaggaa ccctactgag cgtgggcact tgttttaaag caaaactctt
9601 cccaaggact gaagaaaggg cttctggcaa gctcgtcctg gcactgtggt gggatgggtc
9661 tagagtgtca tctgaatggt gcttcctgtg ttcctctttg aattctgcca ttttcagtat
9721 tctcgtgtat ctgaataggc aaagcgattt aattggctgg tcttgcacgc aaattagttc
9781 caaagataag ctctttgtaa cacatttcca gtcgctaatg ctcaaatgta gaacattcct
9841 ttaaatggca ggataaaaaa cccactatcc accatagtgc attttgggaa gatgtctgta
9901 gcatatgttg ctgtgaaatt aggccttgtg ggatatggct gtttgtcatt ttgatgtatt
9961 ttaaataaat atatatattt tttaaagagc cttttttacc agttcaaaaa gtttaattaa
10021 ccagcagtca ccacatctga atttttgtct ctggggcgta gatggcagac caagattaaa
10081 agtggtaact cagccatatg agcatggtct accttcctgg gctgtcctgc tgtcctgcag
10141 agctgctgtt ccgcagacca tgggacacaa ggtcagtgtg ttcccagtga aggtcctaag
10201 tcagtcatct taagtgtttg gtctctgccc cattcagtgg actgttgact tcagtccctg
10261 caagtgcttt agcccgagtg gggttttctc agagcactgc cacaagttaa gtgtgtgttt
10321 agccaaataa tttctccata agggaaaaat gcagtcgccc aaattttacc aacaatgaca
10381 gagatgagag taggaaagat taggcaacat ctgagtttta attggaaaag tgtccaagtc
10441 atcatgaaag gccaactggg agcaagtgat tattagagat tctttaggag acctcatctg
10501 aaaatgttaa gactgccagt gaggaaagga attgttaaaa tgccagcggt tttttttttt
10561 ttctcttttt tttctataat tctgtaaaaa tgcagagaaa gttgagtggt acttcagaat
10621 tgagggagag ggttaccgca gagtagaaat atatttctag atttcagttc caaaccacaa
10681 atccacaaca atgccatttt tcaactgtac aaaaatctgc ttatgaactt gacatgatct
10741 taattggtag tgtcaaaggc caagtttttc acctgttaat atttttccac atttgtcctt
10801 gaatctgaat aactttatac agtactgtaa atttaactta catcaagttt gatgtcaatt
10861 cttatgaaaa gagctttctg catgtaacac atacggttaa agaacacagc aaagaacaaa
10921 atttgcagga acagttttgg aaccaacaga aaatgtcacc ttttatttgc catcttatat
10981 atatatatca gttttaccag ctacttctaa atttgtacat tatttgtaag ggaaagaagg
11041 aaaaccctaa gacttgtcta acttagtgga gaatgtgtgt gttgggctta ggatggatag
11101 ctaagtctta ttgagctgtg ttacctaact tgtatataaa aattgtaatt aaaagtttgg
11161 gttcacctgt ttctcacagt ttaaaatgat gagtaattgc aaattctgga aatgtgacag
11221 tatatgattt aaggctatag aagcaaggaa gctctttcaa gtgctaaaac taaagacttc
11281 tagtttttgg ctcaaattca ataagtactg tttgtatgcc aggatatgtg agatgtaaat
11341 gtagtaggtc acttttcacc cttgtagcta taaaataaaa atttatagaa acagaaatag
11401 cttgtactac tgaattaaca aaagttatac taaagtatca tgttttaaaa atatatatat
11461 atatatacag agttaagctt gttgctgtta ccctgtctgg atttgaaaag tgtgctgatt

11521 tatatatata tattacacac acacacacac acacacacac acacccctaa aatggcctaa
11581 agcagacatc catgtaatta cagttgcaaa atgaaaacat tttggaaaga acattgtatc
11641 atagttcatt catttgcagt ggatctttgt tcctttttac tgtggtaatt ttagaaatga
11701 gtgtcaagtt tgaaattaga tctgctaagt tggggttttg ctgcttgaac tctgcactgg
11761 gtcctcaaat aaaccgatgt gaatgtagtt ttttccccct gtgtgaagaa gcagttacac
11821 cccaacaata ggaggaaaaa tctagaacta tttcaagttt tatctttttg tatatgaaaa
11881 taaaataata ataaaacaa

### Examples

### Experiment Methods and Materials

### Naked Sequences Screening

The ASOs and siRNAs were designed based on the full-length mRNA sequence of ACVR2B (NM_001106.4), and all sequences were derived from the NCBI gene database. All ASOs and siRNAs were designed to ensure homology with the sequences of human (Gene ID: 93, NM_001106.4, SEQ ID NO. 1) and cynomolgus monkey (Gene ID: 102118668, XM_015445674.2, SEQ ID NO. 2).

### Synthesis of Oligonucleotides

The synthesis method for the oligonucleotides is similar to the conventional phosphoramidite solid-phase synthesis method (all were synthesized by Suzhou Beixin Biotechnology Co., Ltd.), which includes four steps: deprotection, coupling, capping, and oxidation or sulfurization. The solid support was used as the starting cycle, and nucleoside monomers were sequentially linked in the 3' to 5' direction. The nucleoside phosphoramidite monomer raw materials, including 2'-OTBDMS, 2'-F RNA, 2'-OMe RNA, etc., were purchased from Shanghai Zhaowei Science and Technology Development Co., Ltd. After completion of solid-phase synthesis, the solid support was transferred to a centrifuge tube, and a 3:1 mixture of 28% ammonium hydroxide and ethanol was added to soak the support at 50 °C for 16 hours to cleave the oligonucleotides from the solid support into solution. After centrifugation, the supernatant was transferred to another centrifuge tube, concentrated to dryness, and then redissolved in deionized water. Purification was performed using a C18 reversed-phase column with 0.1 M TEAA and acetonitrile as the mobile phase. The target oligonucleotide was collected, lyophilized, identified as the desired product by LC-MS, and quantified by UV absorbance at 260 nm. The siRNA was obtained by annealing the sense strand and the antisense strand.

### Dissolution of the compound

The tube containing the compound dry powder was placed in a centrifuge and centrifuged at 12,000 rpm for 5 min. An appropriate amount of RNase-free deionized water was then added, and the mixture was mixed thoroughly to prepare a 10 µM stock solution. The stock solution of each compound was aliquoted into 3 tubes (50 µL per tube) and stored at - 20 °C in a refrigerator.

### Reverse Transfection

Dilution of the compound: The 10 µM stock solution of the compound was diluted with Opti-MEM reduced-serum medium to 12 times the final working concentration and set aside at room temperature. Transfection reagent dilution: The transfection reagent was diluted with Opti-MEM reduced-serum medium at a ratio of 48.5:1.5, gently mixed, and incubated at room temperature for 15 min. Preparation of transfection complexes: Equal volumes of the diluted compound and the diluted transfection reagent were mixed, gently vortexed, and incubated at room temperature for 15 min. Then, 20 µL of the transfection complexes were added to each well of a 96-well cell culture plate. Cell seeding: A-673 cells at approximately 80% confluency were selected, trypsinized, and resuspended in complete medium without penicillin-streptomycin. Cell counting was performed, and a cell suspension at a concentration of 150,000 cells/mL was prepared. Subsequently, 100 µL of the cell suspension was added to each well of the plate containing the transfection complexes. The cells were cultured at 37 °C in a 5% CO₂ incubator for 24 hours.

### Cell Lysis

The cell lysis buffer was prepared according to the table below.

| Reagents | Volume (µl) |
|---|---|
| FlysisAmp cell lysis buffer | 46 |
| DNase I | 2 |
| Enhancer Solution | 2 |

The cell culture plate was taken out, the medium was discarded, and the cells were washed once with pre-chilled DPBS. Then, 50 µL of cell lysis buffer was added to each well, mixed by pipetting 5-10 times, and incubated at room temperature for 10 min. Subsequently, 5 µL of cell lysis stop solution was added to each well, mixed by pipetting 5-10 times, and incubated at room temperature for 2 min to stop the reaction. The cell plate was placed on ice for later use.

### Real-time quantitative PCR detection

### Quantitative PCR reaction Mix

| Reagents | Volume (µl) |
|---|---|
| 5X 1-Step SYBR Green Mix (ROX) | 2 |
| F Primer (10 µM) | 0.2 |
| R Primer (10 µM) | 0.2 |
| Template (cell lysate) | 1 |
| ddH2O (RNase free) | 6.6 |

The quantitative PCR reaction mix was prepared according to the table above, and 9 µL thereof was added to each well of a 384-well plate. Then, 1 µL of cell lysate was added as template. The PCR amplification was performed according to the cycling program shown in the table below.

| Phases | | Temperature | Duration |
|---|---|---|---|
| Pre-denaturation | | 95 °C | 30 s |
| Cycles (40 cycles) | Denaturation | 95 °C | 10 s |
| | Annealing | 60 °C | 30 s |
| Melting Curve | | 95 °C | 15 s |
| | | 60 °C | 60 s |
| | | 95 °C | 15 s |

### Data Analysis

The relative expression levels of genes were analyzed using the relative quantitative PCR method (2^{-ΔΔCt}).

To evaluate the in vitro inhibitory activity of the ASO compounds against ACVR2B mRNA in the A673 cell line, the designed ASO compounds were subjected to in vitro functional evaluation, and the results were presented in Table 3.

**Table 3. In vitro inhibitory activity of ASO compounds against ACVR2B mRNA in the A673 cell line.**

| Compound ID | Average Remaining | | SD | |
|---|---|---|---|---|
| | 500 nM | 100 nM | 500 nM | 100 nM |
| 6001-2 | 0.281 | 0.408 | 0.029 | 0.099 |
| 6003-2 | 0.499 | 0.460 | 0.044 | 0.135 |
| 6005-2 | 0.350 | 0.385 | 0.013 | 0.055 |
| 6009-2 | 0.155 | 0.267 | 0.021 | 0.105 |
| 6011-2 | 0.114 | 0.375 | 0.024 | 0.051 |
| 6012-2 | 0.299 | 0.416 | 0.039 | 0.025 |
| 6017-2 | 0.608 | 0.588 | 0.208 | 0.081 |
| 6023-2 | 0.484 | 0.632 | 0.111 | 0.068 |
| 6026-2 | 0.537 | 0.654 | 0.011 | 0.032 |
| 6027-2 | 0.458 | 0.601 | 0.181 | 0.060 |
| 6030-2 | 0.615 | 0.416 | 0.123 | 0.065 |
| 6037-2 | 0.740 | 0.711 | 0.082 | 0.137 |
| 6043-2 | 0.502 | 0.327 | 0.133 | 0.081 |
| 6049-2 | 0.325 | 0.491 | 0.068 | 0.053 |
| 6050-2 | 0.700 | 0.600 | 0.067 | 0.035 |

To evaluate the *in vitro* inhibitory activity of the siRNA compounds against ACVR2B mRNA in the A673 cell line, the designed siRNA compounds were subjected to *in vitro* functional evaluation, and the results were presented in Table 4.

**Table 4. In vitro inhibitory activity of siRNA compounds against ACVR2B mRNA in the A673 cell line.**

| Compound ID | Average Remaining | | | | | SD | | | | | IC₅₀ |
|---|---|---|---|---|---|---|---|---|---|---|---|
| | 10 nM | 1 nM | 0.1 nM | 0.01 nM | 0.001 nM | 10 nM | 1 nM | 0.1 nM | 0.01 nM | 0.001 nM | nM |
| 6102 | 0.33 | 0.40 | 0.55 | 0.90 | 1.04 | 0.028 | 0.040 | 0.051 | 0.050 | 0.095 | 0.036 |
| 6103 | 0.35 | 0.37 | 0.76 | 1.08 | 0.99 | 0.060 | 0.026 | 0.074 | 0.133 | 0.060 | 0.106 |
| 6104 | 0.43 | 0.40 | 0.42 | 0.90 | 1.01 | 0.042 | 0.066 | 0.051 | 0.075 | 0.095 | 0.014 |
| 6105 | 0.38 | 0.48 | 0.48 | 0.81 | 1.00 | 0.043 | 0.079 | 0.056 | 0.113 | 0.109 | 0.016 |
| 6106 | 0.32 | 0.32 | 0.39 | 0.69 | 1.01 | 0.026 | 0.039 | 0.042 | 0.078 | 0.098 | 0.009 |
| 6107 | 0.29 | 0.35 | 0.67 | 1.14 | 1.01 | 0.036 | 0.020 | 0.029 | 0.157 | 0.114 | 0.098 |
| 6108 | 0.33 | 0.38 | 0.58 | 0.90 | 0.99 | 0.027 | 0.047 | 0.044 | 0.076 | 0.085 | 0.059 |
| 6109 | 0.38 | 0.38 | 0.47 | 0.75 | 0.87 | 0.025 | 0.057 | 0.036 | 0.086 | 0.093 | 0.026 |

To evaluate the in vitro inhibitory activity of the siRNA compounds against ACVR2B mRNA in the A673 cell line, the designed siRNA compounds were subjected to *in vitro* functional evaluation, and the results were presented in Table 5.

**Table 5. In vitro inhibitory activity of siRNA compounds against ACVR2B mRNA in the A673 cell line.**

| Compound ID | Average Remaining | | | SD | | |
|---|---|---|---|---|---|---|
| | 1 nM | 0.1 nM | 0.01 nM | 1 nM | 0.1 nM | 0.01 nM |
| 6104 | 0.50 | 0.53 | 0.58 | 0.06 | 0.14 | 0.12 |
| 6105 | 0.52 | 0.49 | 0.75 | 0.07 | 0.07 | 0.08 |
| 6106 | 0.38 | 0.51 | 0.66 | 0.07 | 0.03 | 0.08 |
| 6109 | 0.49 | 0.59 | 0.64 | 0.14 | 0.17 | 0.06 |
| 6112 | 0.65 | 0.71 | 0.88 | 0.18 | 0.10 | 0.14 |
| 6113 | 0.44 | 0.47 | 0.62 | 0.08 | 0.08 | 0.10 |
| 6115 | 0.47 | 0.48 | 0.61 | 0.05 | 0.03 | 0.05 |
| 6116 | 0.47 | 0.55 | 0.84 | 0.15 | 0.04 | 0.15 |
| 6117 | 0.50 | 0.68 | 0.70 | 0.08 | 0.10 | 0.08 |
| 6119 | 0.49 | 0.67 | 0.74 | 0.03 | 0.22 | 0.17 |
| 6120 | 0.46 | 0.61 | 0.72 | 0.05 | 0.11 | 0.09 |
| 6121 | 0.46 | 0.46 | 0.79 | 0.05 | 0.02 | 0.06 |
| 6122 | 0.53 | 0.56 | 0.60 | 0.07 | 0.05 | 0.12 |
| 6123 | 0.44 | 0.54 | 0.73 | 0.07 | 0.07 | 0.07 |
| 6124 | 0.40 | 0.50 | 0.66 | 0.10 | 0.10 | 0.06 |
| 6125 | 0.51 | 0.67 | 0.69 | 0.09 | 0.14 | 0.11 |
| 6127 | 0.53 | 0.44 | 0.54 | 0.02 | 0.08 | 0.09 |
| 6128 | 0.29 | 0.49 | 0.70 | 0.07 | 0.17 | 0.10 |
| 6129 | 0.71 | 0.71 | 1.26 | 0.17 | 0.07 | 0.05 |
| 6130 | 0.62 | 0.79 | 0.99 | 0.07 | 0.04 | 0.04 |
| 6131 | 0.75 | 0.69 | 0.80 | 0.17 | 0.06 | 0.12 |
| 6132 | 0.47 | 0.70 | 0.98 | 0.09 | 0.11 | 0.09 |
| 6134 | 0.39 | 0.60 | 1.01 | 0.17 | 0.09 | 0.07 |
| 6135 | 0.51 | 0.67 | 1.17 | 0.10 | 0.17 | 0.24 |
| 6137 | 0.44 | 0.48 | 0.73 | 0.06 | 0.07 | 0.08 |
| 6138 | 0.78 | 1.05 | 1.07 | 0.09 | 0.04 | 0.17 |
| 6140 | 0.32 | 0.43 | 0.52 | 0.07 | 0.11 | 0.09 |

## Claims

1. An antisense oligonucleotide comprising an oligonucleotide consisting of 12 to 30 contiguous nucleotides, wherein the oligonucleotide comprises at least 8, at least 9, at least 10, at least 11, at least 12, at least 13, at least 14, at least 15, at least 16, at least 17, at least 18, at least 19, or at least 20 contiguous nucleotides that are at least 80%, 85%, 90%, 95% or 100% complementary to an equal-length contiguous nucleotide sequence of a target region of mRNA encoding activin type IIB receptor (ACVR2B).

2. The antisense oligonucleotide according to claim 1, wherein the target region is positions 504-554, 588-617, 648-686, 837-866, 1234-1268, 1360-1403, 1503-1532, or 1545-1574 of the nucleotide sequence set forth in SEQ ID NO: 1.

3. The antisense oligonucleotide according to claim 1 or 2, wherein the oligonucleotide comprises a nucleotide sequence of at least 8, at least 9, at least 10, at least 11, at least 12, at least 13, at least 14, at least 15, at least 16, at least 17, at least 18, at least 19, or 20 contiguous nucleotides of any one of the nucleotide sequences set forth in SEQ ID NOs: 3 to 17.

4. The antisense oligonucleotide according to any one of claims 1 to 3, wherein when measured over the entire nucleotide sequence of the antisense oligonucleotide, the antisense oligonucleotide has a nucleotide sequence that is at least 80%, at least 85%, at least 90%, at least 95% or 100% complementary to the nucleotide sequence set forth in SEQ ID NO. 1 and/or SEQ ID NO. 2.

5. The antisense oligonucleotide according to any one of claims 1 to 4, wherein antisense oligonucleotide comprises at least one modification selected from a modified sugar moiety, a modified nucleobase moiety, and a modified internucleoside linkage; preferably, the antisense oligonucleotide comprises one or more of the following modifications:
(i) at least one bicyclic sugar moiety, wherein the bicyclic sugar moiety preferably has a 4'-2' bridge, and the 4'-2' bridge is preferably cEt or LNA;
(ii) at least one non-bicyclic modified sugar moiety, preferably selected from 2'-deoxy, 2'-F, 2'-MOE, and 2'-OMe sugar moieties;
(iii) a modified nucleoside, preferably comprising 5-methylcytosine (m5C); and
(iv) at least one sugar surrogate, preferably selected from POM, PNA, THP, and F-HNA.

6. The antisense oligonucleotide according to claim 5, wherein the antisense oligonucleotide comprises a gapmer; preferably, the gapmer comprises: (a) a 5' region consisting of 1 to 6 linked 5' region nucleotides; (b) a central region consisting of 6 to 10 linked central region nucleotides; and (c) a 3' region consisting of 1 to 6 linked 3' region nucleotides; wherein each of the nucleotides in the 5' region and the 3' region comprises a sugar moiety selected from 2'-MOE, LNA, and cEt modified sugar moieties, and at least 6 of the nucleotides in the central region comprise a 2'-deoxy sugar moiety.

7. The antisense oligonucleotide according to claim 6, wherein the antisense oligonucleotide has a sugar motif selected from the group consisting of the following (5' to 3'): eekddddddddddkke, ekkddddddddddkke, kkkdyddddddddkkk, kkkddydddddddkkk, kkkdddyddddddkkk, kkkddddddddddkkk, and eeeeeeddddddddddeeeee; wherein e represents a 2'-MOE sugar moiety, k represents a cEt sugar moiety, d represents a 2'-deoxy sugar moiety, and y represents a 2'-OMe sugar moiety.

8. The antisense oligonucleotide according to any one of claims 1 to 7, wherein the antisense oligonucleotide comprises at least one modified internucleoside linkage; preferably, each internucleoside linkage of the antisense oligonucleotide is a modified internucleoside linkage; preferably, the modified internucleoside linkage is selected from phosphorothioate (PS) and phosphoramidate (PN) (e.g., methanesulfonyl phosphoramidate (MsPA)), preferably PS.

9. The antisense oligonucleotide according to any one of claims 1 to 8, wherein the antisense oligonucleotide comprises an oligonucleotide consisting of 12-30, 14-22, 14-20, 14-18, 14-20, 15-17, 15-25, or 16-20 linked nucleotides, preferably, the oligonucleotide consists of 20 contiguous nucleotides; preferably, the antisense oligonucleotide comprises, or consists of, a nucleotide sequence as set forth in any one of SEQ ID NOs: 18 to 32, or a nucleotide sequence having 1 to 3 nucleotide differences therefrom.

10. The antisense oligonucleotide according to any one of claims 1 to 9, wherein the antisense oligonucleotide comprises a ligand; preferably, wherein the ligand comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; or the ligand comprises a ligand targeting non-hepatocyte cells, preferably the ligand is a lipophilic group, an integrin ligand, or a transferrin receptor 1 ligand; the lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

11. An RNAi agent for inhibiting expression of the activin type IIB receptor (ACVR2B) gene in a cell, comprising a sense strand and an antisense strand forming a duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding ACVR2B, or a nucleotide sequence having no more than 3 nucleotide differences therefrom.

12. The RNAi agent according to claim 11, wherein the target region is positions 508-534, 634-660, 655-695, 733-774, 756-782, 929-955, 1049-1089, 1141-1180, 1189-1215, 1243-1269, 1276-1321, 1377-1403, 1547-1596, 1642-1690, 1744-1773, 1916-1942, or 1949-1975 of the nucleotide sequence set forth in SEQ ID NO: 1.

13. The RNAi agent according to claim 11 or 12, wherein the antisense strand comprises at least 15 contiguous nucleotides of a nucleotide sequence selected from SEQ ID NOs: 64 to 94 and nucleotide sequences having 1 to 3 nucleotide differences therefrom.

14. The RNAi agent according to any one of claims 11 to 13, wherein the duplex region is 17 to 23 base pairs in length, preferably 18 to 21 base pairs, more preferably 19 base pairs.

15. The RNAi agent according to any one of claims 11 to 14, wherein each of the sense strand and the antisense strand is 17 to 23 nucleotides in length, preferably 19 to 21 nucleotides.

16. The RNAi agent according to any one of claims 11 to 15, wherein the RNAi agent comprises one or two blunt ends, preferably one blunt end.

17. The RNAi agent according to any one of claims 11 to 16, wherein the RNAi agent comprises one or two overhangs, preferably one overhang, each overhang comprising 1 to 4 unpaired nucleotides, preferably 2 unpaired nucleotides.

18. The RNAi agent according to claim 17, wherein the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

19. The RNAi agent according to any one of claims 11 to 18, wherein the sense strand comprises at least 15 contiguous nucleotides of any nucleotide sequence selected from SEQ ID NOs: 33 to 63 and nucleotide sequences having 1 to 3 nucleotide differences therefrom.

20. The RNAi agent according to any one of claims 11 to 19, wherein the antisense strand has no more than 23 nucleotides and comprises a nucleotide sequence selected from SEQ ID NOs: 64 to 94; and the sense strand has no more than 21 nucleotides and comprises a nucleotide sequence from SEQ ID NOs: 33 to 63.

21. The RNAi agent according to claim 20, wherein
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:79 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:80 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:81 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:82 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:83 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:84 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:85 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:86 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:87 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:88 or a nucleotide sequence having 1, 2, or 3 nucleotide differences therefrom;
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:89 or a nucleotide sequence having 1, 2, or 3 nucleotide differences therefrom;
the sense strand comprises or consists of a sequence set forth in SEQ ID NO:59 or a nucleotide sequence having 1, 2, or 3 nucleotide differences therefrom, and the antisense strand comprises or consists of a sequence set forth in SEQ ID NO:90 or a nucleotide sequence having 1, 2, or 3 nucleotide differences therefrom;
the sense strand comprises or consists of a sequence set forth in SEQ ID NO:60 or a nucleotide sequence having 1, 2, or 3 nucleotide differences therefrom, and the antisense strand comprises or consists of a sequence set forth in SEQ ID NO:91 or a nucleotide sequence having 1, 2, or 3 nucleotide differences therefrom;
the sense strand comprises or consists of a sequence set forth in SEQ ID NO:61 or a nucleotide sequence having 1, 2, or 3 nucleotide differences therefrom, and the antisense strand comprises or consists of a sequence set forth in SEQ ID NO:92 or a nucleotide sequence having 1, 2, or 3 nucleotide differences therefrom;
the sense strand comprises or consists of a sequence set forth in SEQ ID NO:62 or a nucleotide sequence having 1, 2, or 3 nucleotide differences therefrom, and the antisense strand comprises or consists of a sequence set forth in SEQ ID NO:93 or a nucleotide sequence having 1, 2, or 3 nucleotide differences therefrom; or
the sense strand comprises or consists of a sequence set forth in SEQ ID NO:63 or a nucleotide sequence having 1, 2, or 3 nucleotide differences therefrom, and the antisense strand comprises or consists of a sequence set forth in SEQ ID NO:94 or a nucleotide sequence having 1, 2, or 3 nucleotide differences therefrom.

22. The RNAi agent according to claim 21, wherein the RNAi agent comprises duplex 6102, 6103, 6104, 6105, 6106, 6107, 6108, 6109, 6112, 6113, 6115, 6116, 6117, 6119, 6120, 6121, 6122, 6123, 6124, 6125, 6127, 6128, 6129, 6130, 6131, 6132, 6134, 6135, 6137, 6138, or 6140.

23. The RNAi agent according to any one of claims 11 to 22, wherein the sense strand and/or antisense strand of the RNAi agent comprises at least one modified nucleotide; the modified nucleotide is independently selected from the group consisting of: 2'-deoxythymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

24. The RNAi agent according to claim 23, wherein in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

25. The RNAi agent according to claim 23 or 24, wherein the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

26. The RNAi agent according to any one of claims 23 to 25, wherein in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

27. The RNAi agent according to any one of claims 23 to 26, wherein the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

28. The RNAi agent according to claim 23, wherein the modification is selected from one of STC, ESC, Advanced ESC, ESC+, AD1-3, AD5 and GalXC.

29. The RNAi agent according to any one of claims 23 to 28, wherein the 5'-terminal nucleotide of the antisense strand contains a phosphate or phosphate analog modification, preferably 5'-VP.

30. The RNAi agent according to any one of claims 23 to 29, wherein the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; alternatively, the RNAi agent comprises a ligand targeting non-hepatocyte cells, preferably a lipophilic group, an integrin ligand, or a transferrin receptor 1 ligand; the lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

31. A pharmaceutical composition comprising an antisense oligonucleotide according to any one of claims 1 to 10, or an RNAi agent according to any one of claims 11 to 30; and a pharmaceutically acceptable carrier; preferably, the pharmaceutical composition is formulated for intravenous or subcutaneous injection.

32. Use of an antisense oligonucleotide according to any one of claims 1 to 10, an RNAi agent according to any one of claims 11 to 30, or a pharmaceutical composition of claim 31 in the manufacturing of a medicament for:
(i) reducing expression level of ACVR2B in a cell;
(ii) preventing or treating a disease caused by an elevated ACVR2B expression level; or
(iii) preventing or treating a disease selected from muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.

33. Use of an agent that reduces expression of the gene encoding activin type IIB receptor (ACVR2B) in the manufacture of a medicament for the treatment of a disease or condition treatable by ACVR2B knockdown; preferably, the agent is an antisense oligonucleotide and/or an RNAi agent; preferably, the disease or condition is selected from muscle atrophy, muscular dystrophy, obesity, diabetes mellitus, chronic kidney disease, acute kidney injury, post-traumatic or postoperative healing impairment, maxillofacial tumors, preeclampsia, liver fibrosis, atherosclerosis, ischemic stroke, and Alzheimer's disease.
